(19) Europäisches Patentamt
European Patent Office
Office européen des brevets

(11) EP 2 124 926 B1

(12) **EUROPEAN PATENT SPECIFICATION**

(45) Date of publication and mention of the grant of the patent:
**25.09.2013 Bulletin 2013/39**

(21) Application number: **08701950.1**

(22) Date of filing: **25.01.2008**

(51) Int Cl.:
***A61K 31/216*** *(2006.01)* ***A61K 31/24*** *(2006.01)*
***A61K 31/245*** *(2006.01)* ***A61P 11/00*** *(2006.01)*
***A61P 11/12*** *(2006.01)*

(86) International application number:
**PCT/GB2008/000277**

(87) International publication number:
**WO 2008/090366 (31.07.2008 Gazette 2008/31)**

(54) **Treatment for respiratory disease**

Behandlung von Atemwegserkrankungen

Traitement pour maladie respiratoire

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **25.01.2007 GB 0701456**

(43) Date of publication of application:
**02.12.2009 Bulletin 2009/49**

(73) Proprietor: **Mucokinetica Ltd.**
**London SW12 0BL (GB)**

(72) Inventors:
- **HALL, Roderick, Lindsay**
  **London SW12 0BL (GB)**
- **COLE, Peter, John**
  **London SW12 0BL (GB)**

(74) Representative: **Baldock, Sharon Claire**
**Boult Wade Tennant**
**Verulam Gardens**
**70 Gray's Inn Road**
**London WC1X 8BT (GB)**

(56) References cited:
**EP-A- 0 048 433**
**EP-A- 0 350 840**
**EP-A- 0 893 437**
**EP-A- 1 314 779**
**WO-A-03/070235**
**WO-A-2004/022096**
**WO-A-2006/108643**

- **CHEN ET AL: "Serine protease inhibitors nafamostat mesilate and gabexate mesilate attenuate allergen-induced airway inflammation and eosinophilia in a murine model of asthma" JOURNAL OF ALLERGY AND CLINICAL IMMUNOLOGY, MOSBY - YEARLY BOOK, INC, US, vol. 118, no. 1, 1 July 2006 (2006-07-01), pages 105-112, XP005610110 ISSN: 0091-6749**
- **UCHIBA MITSUSHIRO ET AL: "Effect of nafamostat mesilate on pulmonary vascular injury induced by lipopolysaccharide in rats" AMERICAN JOURNAL OF RESPIRATORY AND CRITICAL CARE MEDICINE, vol. 155, no. 2, 1997, pages 711-718, XP008093420 ISSN: 1073-449X**
- **DATABASE WPI Week 200438 Derwent Publications Ltd., London, GB; AN 2004-411425 XP002485138 & WO 2004/041309 A (ONO PHARM CO LTD) 21 May 2004 (2004-05-21)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Field of Invention

The current invention relates to a new medical use of a family of amidino compounds in the treatment of respiratory diseases characterised by poor mucociliary clearance or mucostasis.

In a preferred embodiment the invention relates to the use of 6- amidino- 2- napthyl 4- guanidinobenzoate in the manufacture of a medicament for the treatment of said respiratory diseases.

### Background

In the respiratory epithelium the properties and composition of the airway surface liquid (ASL) that lines the airway surfaces are critical to effective operation of airway defence functions, especially mucociliary clearance. Absorption of sodium ions via amiloride-sensitive sodium channels (ENaC) located at the apical surface of epithelial cells is particularly important in regulating mucociliary function in health and disease. Pharmacological treatments applied to the airway epithelium to lower ENaC activity also act to increase mucociliary clearance activity. Mucostasis in respiratory disease (i.e. accumulation of secretions and defective clearance) is an important untreated medical need. Poor clearance of secretions from the lower airways is a continuing problem and is believed to lead to chronic infection, inflammation in the airways, reduction in lung function and in some conditions to destruction of the lungs and eventually to respiratory failure. Lowering ENaC activity is a promising strategy to improve performance of mucociliary clearance in respiratory diseases characterised by mucostasis; cystic fibrosis (CF); bronchiectasis; chronic bronchitis; chronic obstructive pulmonary disease (COPD); rhino-sinusitis; otitis media. An effective treatment to enhance mucociliary clearance is thought to have potential to achieve significant benefit for respiratory disease including diseases of the lung, airways (bronchioles, bronchi), and upper respiratory tract (nose, para-nasal sinuses, Eustachian tube and middle ear).

Amiloride and analogues have been evaluated in the clinic as potential inhaled therapies for treatment of respiratory diseases characterised by mucostasis, particularly CF. Although amiloride is a potent blocker of ENaC the clinical results have been disappointing. Rapid reversibility of binding to ENaC together with rapid absorption by the airway epithelium are thought to be responsible for the short duration of efficacy achieved in patients.

Amiloride is a potent, but short acting blocker of the epithelial sodium channel, where reduction of ENaC activity is achieved by direct exofacial block in the pore of the channel. Washing epithelial cells to remove amiloride results in rapid recovery of ENaC activity, typically within 1 to 2 minutes.

Down regulation of airway ENaC activity has also been achieved by Kunitz-type serine protease inhibitors, aprotinin and BAY 39-9437 (Bridges et al 2001). The proposed mechanism of action of the Kunitz inhibitors is to inhibit (an) endogenous protease(s) responsible for proteolytic activation of sodium channel proteins in the apical membrane of epithelial cells. By contrast to the short acting blocker profile of amiloride, BAY 39-9437 is an example of a long duration of action down regulator of ENaC. Treatment of airway epithelial cells with BAY 39-9437 results in reduction of ENaC activity that is sustained for an extended period of time after unbound substance has been removed, by washing, from contact with the epithelial cells (Bridges et al 2001).

Potent, long duration down regulators of ENaC activity are potentially efficacious inhaled treatments for respiratory diseases characterised by mucostasis.

6- amidino- 2- napthyl 4- guanidinobenzoate, was first described in Japanese patent no. JP57053454. It is also described in United States patent no. US4454338 as a member of a family of related amidino compounds, which are described as having powerful antitrypsin, antiplasmin, antikallikrein, antithrombin, antigranzyme and anticompliment activity. 6- amidino- 2- napthyl 4- guanidinobenzoate is also known to act as an anti- inflammatory agent (Iwaki et al 1984) and an anticoagulant (Hitomi et al 1985) . EP 1314779 discloses the mucus secretion promotion action of Nafamostat.

The substance has been approved for use in the treatment of the acute symptoms of pancreatitis, disseminated intravascular coagulation and patients with bleeding complications or tendency to bleed. It has also been used to prevent blood coagulation during extracorporeal blood circulation, for example in hemodialysis and plasmapheresis. Muto et al. (1993) reported that 6- amidino- 2- napthyl 4- guanidinobenzoate perfused at a high dose of $10^{-4}$ M in the cortical collecting duct of the rabbit kidney caused a change in transepithelial voltage equivalent to 40% of that achievable by $5.10^{-6}$ M amiloride. The 10- fold lower concentration of $10^{-5}$ M 6- amidino- 2- napthyl 4- guanidinobenzoate was without effect on the rabbit kidney model. It should be noted that the minimum effective concentration in the kidney model is 1000- fold higher than the minimum significant concentration that achieves down regulation of ENaC activity in the human airway epithelial cell.

The profile of the change in transepithelial voltage in response to perfusion of $10^{-4}$ M 6- amidino- 2- napthyl 4- guanidinobenzoate (Muto et al. 1993) shows rapid onset within 30s of perfusion, and rapid recovery within 1 minute to starting voltage when perfusion is stopped. This profile is similar to that observed for perfusion of amiloride in the model.

It is known that after intravenous infusion of 6- amidino- 2- napthyl 4- guanidinobenzoate in animals and man that the metabolites 4- guanidinobenzoic acid (pGBA) and amidino- 2- napthol (AN) are excreted into the urine via the kidney cortical collecting duct.

In a later paper (Muto et al. 1994) the same authors investigated the possibility that the observed effects in the kidney model could be achieved by the metabolites. They reported (Muto et al. 1994) that pGBA was active when perfused at $10^{-5}$ M, 10- fold lower than the minimum effective concentration for 6- amidino- 2- napthyl 4- guanidinobenzoate. AN was also active but the minimum effective concentration was again $10^{-4}$ M (Muto et al. 1994) . Muto et al. (1993; 1994) report that in the kidney model 6- amidino- 2- napthyl 4- guanidinobenzoate may act as a weak, short acting inhibitor of the kidney sodium channel, and that the effect may be mediated by the metabolites of 6- amidino- 2- napthyl 4- guanidinobenzoate, particularly pGBA that was 10- fold higher potency than 6- amidino- 2- napthyl 4- guanidinobenzoate. The kidney model results do not predict potent, long duration down regulation of airway ENaC activity sustained at least 1h 45 min. after washout from contact with doses up to 1000- fold below the minimum effective concentration in the kidney model.

Rossier (2004) reviewed hormonal and serine protease regulation of the epithelial sodium channel. There are clear differences in hormonal control of the activity of ENaC in different organs and tissues. In the kidney and colon ENaC expression is under mineralocorticoid (aldosterone) control, whereas in the distal lung glucocorticoid control operates.

The first report (Vallet et al. 1997) of a serine protease activating ENaC was channel activating protease 1 (CAP-1) in *Xenopus* oocytes - an amphibian egg model. Since the first report at least two further channel activating proteases (CAP-2 & 3) have been reported with homologues in mammalian species, including human (see Rossier, 2004). Involvement of CAP homologues in regulation of ENaC activity in mammalian epithelial systems, including the kidney and the airway, has been reported. It is currently not known if regulation of ENaC activity in mammalian systems is controlled by activity of individual CAP homologues, or by mechanisms involving a combination of two, or a cascade of all three.

The mammalian homologue of CAP- 1 is prostasin. Prostasin expressed in the membrane of human airway epithelial cells has been implicated (Tong et al. 2004) to have an involvement in protease regulation of ENaC in airway epithelial cells. The contribution of homologues of the other CAPs, or other serine proteases to the normal and pathological regulation of ENaC in the airways is not known at this time. There are no reports that 6- amidino- 2- napthyl 4- guanidinobenzoate is an inhibitor of the enzymic catalytic activity of CAPs or CAP homologues, including prostasin.

Differences have been reported in the details of the protease regulation mechanisms in different mammalian epithelia and organs. Consistent with mineralocorticoid regulation of ENaC expression in the kidney, Narikiyo et al. (2002) found that aldosterone also increased prostasin expression in the mouse kidney cell line M-1, and also that urinary excretion of prostasin was increased approximately 4-fold when rats were intravenously infused with aldosterone.

Differences in cellular expression of prostasin have also been noted. Full length prostasin is a glycosylphosphatidylinositol (GPI)- membrane anchored protein. Tong et al. (2004) reported that in three human airway epithelial cell lines, JME/CF15, Calu- 3, and A549, prostasin was expressed as a membrane anchored protein with little secreted into the medium in which the cells were cultured. By contrast Iwashita et al. (2003) observed that for the mouse kidney cell line, M- 1 cells, prostasin was found secreted in the culture medium, and <u>not</u> in the membrane fraction of the cells as GPI- membrane anchored prostasin, nor was prostasin detected in the cell cytosol fraction. Iwashita et al. (2003) also reported detection of prostasin in the urine of rats and humans, and being unable to identify prostasin in cytosolic or membrane fractions prepared from rat kidneys. They postulate that in both M- 1 cells and in the kidneys prostasin is expressed as a secretory protein, rather than as a membrane bound protein as observed for airway cells.

Iwashita et al. (2003) reported that treatment of M- 1 kidney cells with 6- amidino- 2- napthyl 4- guanidinobenzoate for 24h reduced prostasin secretion into culture medium; also rats infused i.v. with 6- amidino- 2- napthyl 4- guanidinobenzoate for 24h showed reduced levels of prostasin secreted in the urine. However, given the clear protein expression differences for prostasin between airway epithelial cells and kidney, it is not apparent how, or if, 6- amidino- 2- napthyl 4- guanidinobenzoate would affect prostasin expression or ENaC activity in airway epithelial systems, and at time periods much shorter than the 24h studied in the kidney models.

The current inventors are therefore unaware of any previous use of 6- amidino- 2- napthyl 4- guanidinobenzoate or related compounds to regulate the activity of ENaC in airway epithelial cells or the respiratory system in general.

Surprisingly, the current inventors have found 6- amidino- 2- napthyl 4- guanidinobenzoate to be a potent long duration inhibitor of ENaC activity in airway epithelial cells. This and related compounds therefore have potential in the treatment of respiratory diseases characterised by mucostasis such as cystic fibrosis (CF) ; bronchiectasis; chronic bronchitis; chronic obstructive pulmonary disease (COPD) and rhino- sinusitis.

## <u>Statement of Invention</u>

In a first aspect the present invention is directed to the use of an amidino compound of the general formula (I):

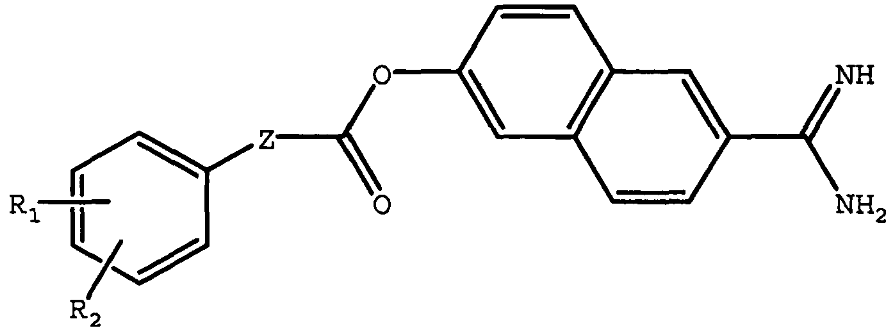

or a pharmaceutically acceptable salt thereof, in the manufacture of a medicament for the treatment of respiratory diseases characterised by poor mucociliary clearance or mucostasis.

In formula (I), Z represents - $(CH_2)_a$-,

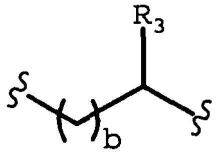 , 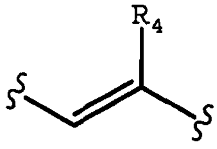 or 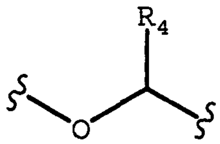

wherein a is 0, 1, 2 or 3, b is 0, 1 or 2, $R_3$ is a straight or branched chain alkyl group of 1 to 4 carbon atoms or a cycloalkyl group of 3 to 6 carbon atoms, $R_4$ is a hydrogen atom or a straight or branched chain alkyl group of 1 to 4 carbon atoms.

$R_1$ and $R_2$, which may be the same or different, represent each a hydrogen atom, a straight or branched chain alkyl group of 1 to 4 carbon atoms, -O- $R_5$, -S- $R_5$, -$COOR_5$, -$COR_6$, -O- $COR_7$, -$NHCOR_7$,

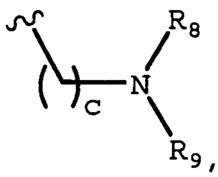 , 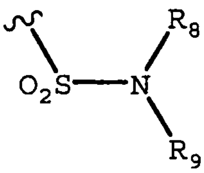 , 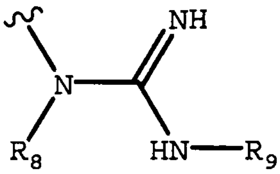

$NO_2$, CN, halogen, $CF_3$, methylenedioxy, or

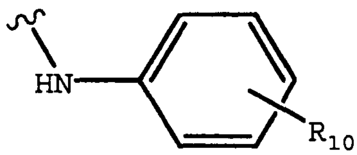

wherein c is 0, 1 or 2; $R_5$ is a hydrogen atom, linear or branched chain alkyl group of 1 to 4 carbon atoms, or benzyl group; $R_6$ is a hydrogen atom or straight or branched chain alkyl group of 1 to 4 carbon atoms; $R_7$ is a straight or branched chain alkyl group of 1 to 4 carbon atoms; $R_8$ and $R_9$, which may be the same or different, are each a hydrogen atom, straight or branched chain alkyl group of 1 to 4 carbon atoms, or amino radical protecting group; and $R_{10}$ is a hydrogen atom, methyl or $CF_3$. The straight or branched chain alkyl groups of 1 to 4 carbon atom include methyl, ethyl, n- propyl, isopropyl, n- butyl, isobutyl and tert- butyl. The medicament is an aqueous solution with a chloride concentration of less than 10mM.

Examples of $R_1$ and $R_2$ include hydrogen, methyl, ethyl, n- propyl, n- butyl, tert- butyl, hydroxy, methoxy, ethoxy, n- propyloxy, n- butyloxy, benzyloxy, mercapto, methylthio, ethylthio, carboxy, methoxycarbonyl, ethoxycarbonyl, butoxycarbonyl, formyl, acetyl, ethylcarbonyl, guanidino, N- methylguanidino, N- n- butylguanidino, acetoxy, propionyloxy, butyryloxy, acetamino, propionyl amino, butyrylamino, amino, dimethylamino, dibutylamino, aminomethyl, benzyloxycarbonylaminomethyl, sulfamyl, dimethylsulfamyl, nitro, cyano, fluorine, chlorine, bromine, iodine, trifluoromethyl, methylenedioxy, phenylamino, 3, 4- dimethylphenylamino, and 3- trifluoromethylphenylamino.

In preferred embodiments Z represents a covalent bond.

Preferably $R_1$ or $R_2$ represent hydrogen or a straight or branched alkyl group having from 1 to 4 carbon atoms.

In particularly preferred embodiments $R_1$ is hydrogen and $R_2$ is

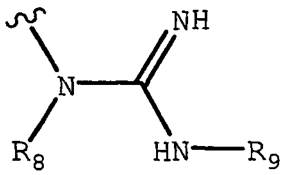

wherein $R_8$ and $R_9$ are hydrogen.

Preferably, the compound will be in the form of a pharmaceutically acceptable salt or ester.

The medicament may optionally further comprise one or more pharmaceutically acceptable carriers, excipients or diluents.

Preferably, the medicament in aqueous solution of low chloride concentration further comprises a pharmaceutically acceptable impermeant, monovalent anion in the range of from 100mM to 160mM, preferably in the range of from 140mM to 150mM. Preferably the pharmaceutically acceptable impermeant, monovalent anion is gluconate.

Most preferably, the compound is 6- amidino- 2- napthyl 4- guanidinobenzoate dihydrochloride or a pharmaceutically acceptable salt or ester thereof. Examples include 6- amidino- 2- napthyl 4- guanidinobenzoate dihydrochloride and 6- amidino- 2- napthyl 4- guanidinobenzoate mesylate.

In preferred embodiments the amidino compound according to formula I is the sole active ingredient.

The intended respiratory diseases include diseases of the lung, airways (bronchioles, bronchi), and upper respiratory tract (nose, para-nasal sinuses).

Preferably the disease is selected from the group comprising cystic fibrosis (CF); bronchiectasis; chronic bronchitis; chronic obstructive pulmonary disease (COPD); rhino-sinusitis.

Preferably, the respiratory disease is cystic fibrosis.

Some embodiments of the medicaments of the invention include other active agents selected from the group of antibiotics, vaccines, decongestants (nasal or bronchial), mucolytic agents, rhDNase, non-steroidal antiinflamatory agents (NSAIDs), steroids, antiviral agents, elastase inhibitors, exofacial sodium channel blocking agents, gene therapy agents, chloride channel activators, mannitol, purinergic receptor agonists and bronchodilators.

In one embodiment of the invention, the medicament is formulated for administration to the respiratory system by the pulmonary route.

Preferably, the medicament is formulated for administration by a method including intratracheal instillation (delivery of solution into the airways by syringe), intratracheal delivery of liposomes, insufflation (administration of powder formulation by syringe or any other similar device into the airways), nebulization, and aerosol inhalation.

Aerosols (e.g. , jet or ultrasonic nebulizers, metered-dose inhalers (MDIs) can also be used in intranasal applications as well as for pulmonary administration.

Aerosol formulations are stable dispersions or suspensions of solid material and liquid droplets in a gaseous medium and can be placed into pressurized acceptable propellants, such as hydrofluoroalkanes (HFAs, i.e. HFA-134a and HFA-227, or a mixture thereof), dichlorodifluoromethane (or other chlorofluorocarbon propellants such as a mixture of Propellants 11,12, and/or 114), propane, nitrogen, and the like.

Also described herein is a formulation for administration by dry powder inhalation or aerosol inhalation together with a propellant selected from the group comprising hydrofluoroalkanes, chlorofluorocarbons, propane, nitrogen, or a mixture thereof.

Also disclosed are medicaments formulated for the treatment of the nasal epithelium, para-nasal sinuses, the Eustachian tube and middle ear, and are formulated as solutions for delivery as drops, by pipette or by syringe to the nasal epithelium, para-nasal sinuses, the Eustachian tube and middle ear.

For the treatment of rhino-sinusitis, the medicament is formulated as a solution for the irrigation of the paranasal sinuses by local instillation of said solution via cannula tube or syringe needle.

For the treatment of otitis media, the medicament may be formulated as a solution for delivery as solution directly applied to the middle ear via the external auditory meatus and canal.

Also described herein is a method of treating respiratory disease, comprising administration of a therapeutically effective amount of an amidino compound formula I to a patient in need thereof.

Preferably, the compound will be in the form of a pharmaceutically acceptable salt or ester.

More preferably, the compound is administered together with one or more pharmaceutically acceptable carriers or diluents.

In certain situations, the compound is administered as an aqueous solution.

The aqueous solution may have a low concentration of chloride. Preferably, the total chloride concentration administered is less than 10mM.

Preferably, the amidino compound in aqueous solution of low chloride concentration is administered together with a pharmaceutically acceptable impermeant, monovalent anion with a concentration in the range of from 100mM to 160mM, preferably in the range of from 140mM to 150mM. Preferably, the pharmaceutically acceptable impermeant, monovalent anion is gluconate.

Most preferably, the compound is 6- amidino- 2- napthyl 4- guanidinobenzoate dihydrochloride or a pharmaceutically acceptable salt or ester thereof. Examples include 6- amidino- 2- napthyl 4- guanidinobenzoate dihydrochloride and 6- amidino- 2- napthyl 4- guanidinobenzoate mesylate.

Preferably, the respiratory disease to be treated is characterised by poor mucociliary clearance and/or mucostasis.

Preferably, the disease is selected from the group comprising cystic fibrosis (CF) ; bronchiectasis; chronic bronchitis; chronic obstructive pulmonary disease (COPD) ; rhino- sinusitis and otitis media. Most preferably, the respiratory disease is cystic fibrosis.

Preferably the administration is to the respiratory system by the pulmonary route.

Such an administration may be achieved by intratracheal instillation (delivery of solution into the airways by syringe), intratracheal delivery of liposomes, insufflation (administration of powder formulation by syringe or any other similar device into the airways), nebulization, dry powder inhalation and aerosol inhalation. Most preferably, the administration is by dry powder inhalation or aerosol inhalation together with a propellant selected from the group comprising hydrofluroalkanes, chlorofluocarbons, propane, nitrogen, or a mixture thereof.

Also described herein is the use of amidino compounds of formula I in the treatment of conditions benefiting from lowered airway epithelial sodium channel activity.

Also described herein is the use of amidino of formula I in the treatment of conditions benefiting from increased ciliary transport in the airways of a patient.

Also described herein are pharmaceutical compositions comprising amidino compounds of Formula I, formulated for delivery to the respiratory system.

Preferably, the amidino compounds comprised in the pharmaceutical compositions are in the form of a pharmaceutically acceptable salt or ester.

More preferably, the pharmaceutical compositions also comprise one or more pharmaceutically acceptable carriers or diluents.

In certain embodiments, the pharmaceutical composition is in the form of an aqueous solution.

In certain embodiments of the pharmaceutical composition in aqueous solution, the pharmaceutical composition has a low concentration of chloride. Preferably, the pharmaceutical composition has a chloride concentration of less than 10mM.

Preferably, the pharmaceutical composition in aqueous solution of low chloride concentration further comprises a pharmaceutically acceptable impermeant, monovalent anion in the range of from 100mM to 160mM, preferably in the range of from 140mM to 150mM. Preferably, the pharmaceutically acceptable impermeant, monovalent anion is gluconate.

Most preferably, the amidino compound comprised in the pharmaceutical compositions of the invention is 6-amidino- 2- napthyl 4- guanidinobenzoate or a pharmaceutically acceptable salt or ester thereof.

The pharmaceutical compositions may be formulated for administration to the respiratory system by the pulmonary route.

Preferably, the pharmaceutical compositions are formulated for administration by any one of intratracheal instillation, intratracheal delivery of liposomes, insufflation, nebulization, dry powder inhalation and aerosol inhalation.

Most preferably, the pharmaceutical compositions are formulated for administration by dry powder inhalation or aerosol inhalation and further comprise a propellant selected from the group comprising hydrofluroalkanes, chlorofluocarbons, propane, nitrogen, or a mixture thereof

The current invention further relates to an inhalation , device loaded with a pharmaceutical composition described herein. Preferably, the inhalation device is a metered dose inhaler, jet nebulizer or ultrasonic nebulizer.

## Description of the Figures

The invention will be now be described by way of exemplification only and with reference to the accompanying figures, brief descriptions of which follow:

### Figure 1:

Short circuit current measurement of human bronchial epithelial (HBE) cells 24d in culture, 21d air-liquid interface (ALI). No pre-treatment prior to mounting in Ussing chamber. At 29min following assembly of the chamber the basolateral and apical sides were flushed through each with 30ml of fresh Krebs-Ringer buffer whilst maintaining the volume on

each side of the chamber at 5ml buffer. At 24.5min 5μl of $10^{-2}$ M-amiloride (final concentration 10 μM) was added to the apical chamber. At 36min were flushed as previously with 30ml of fresh Krebs-Ringer buffer. $\Delta I_{sc}$ (amiloride addition) = 9.2 μAmp

$$R_T = 1092 \pm 64\ \text{Ohm/cm}^2$$

**Figure 2:**

Short circuit current recordings of HBE cells 24d (A&B) & 25d (C&D) in culture, 19 & 20d ALI. A & C: Controls. B & D: Pre- treated with $10^{-6}$ M 6- amidino- 2- napthyl 4- guanidinobenzoate for 90min prior to assembly in Ussing chambers and measurement of $I_{sc}$. Amiloride to a final concentration of 10μM was added to the apical surface of cells at 30min after washing of cells with Krebs- Ringer buffer and assembly in Ussing chambers (approximately 10min from start of recording) . Forskolin to a final concentration of 10μM was added to both the apical and basolateral surface 7min later.

**Figure 3:**

A: Amiloride $\Delta I_{sc}$, and B: Forskolin $\Delta I_{sc}$, from short circuit current recordings from HBE cells cultured for 24 & 25d, 19 & 20d at ALI. Results are studies for two culture batches. Closed circles: Cultures treated and incubated with 1μM 6- amidino- 2- napthyl 4- guanidinobenzoate for 90min. prior to wash with Krebs- Ringer buffer and assembly into Ussing chambers. Open circles: Non- treatment controls. Amiloride 10μM final concentration was added to the apical surface 30min. after Krebs- Ringer buffer wash and assembly of Ussing chambers. Forskolin was added apically and basolaterally at 37min.

**Figure 4:**

Dose- response study: Short circuit current response to 90min treatment and incubation with 6- amidino- 2- napthyl 4- guanidinobenzoate over dose range $3.10^{-6}$M to $3.10^{-8}$M (closed bars) for HBE cells cultured for 18 & 19d, 15 & 16d at ALI. Non- treatment controls (open bars) . Amiloride 10μM final concentration was added to the apical surface 30min. after Krebs- Ringer buffer wash and assembly in Ussing chambers. Forskolin was added apically and basolaterally at 37min. A: Amiloride $\Delta I_{sc}$ (mean±SEM), and B: Forskolin $\Delta I_{sc}$ (mean±SEM) from short circuit current recordings. Controls and treatment groups analysed by one- way ANOVA. Tukey's multiple comparison test used to further analyse amiloride $\Delta I_{sc}$ results.

**Figure 5:**

Time of onset study: Amiloride $\Delta I_{sc}$ response to treatment and incubation with $10^{-6}$ M 6- amidino- 2- napthyl 4- guanidinobenzoate over time range 5 min. to 60 min. (closed circles) for HBE cells cultured for 22d, 19d at ALI. Non- treatment controls (open circles) . Amiloride 10μM final concentration was added to the apical surface 30min. after Krebs- Ringer buffer wash and assembly in Ussing chambers. Controls and treatment groups analysed by one- way ANOVA. Tukey's multiple comparison test used to compare groups.

**Figure 6:**

Duration of amiloride $\Delta I_{sc}$ response : Amiloride $\Delta I_{sc}$ response of HBE cells cultured for 26d, 23d at ALI, treated with $10^{-6}$ M 6- amidino- 2- napthyl 4- guanidinobenzoate for 60 min. (closed circles), followed by periods of washout with fresh culture medium: 0min, 1h15min, 3h00min and 4h30min. Non- treatment controls (open circles) . Amiloride 10μM final concentration was added to the apical surface 30min. after Krebs- Ringer buffer wash and assembly in Ussing chambers. Controls and treatment groups analysed by one- way ANOVA. Tukey's multiple comparison test used to compare groups.

**Figure 7:**

Short circuit current recordings of HBE cells 26 & 27d in culture, 23 & 24d ALI. A: Controls. B to E: Treated with $10^{-6}$M 6- amidino- 2- napthyl 4- guanidinobenzoate for 60min, followed by a period of washout with fresh culture medium for- B: 0min. C: 1h 15min. D: 3h 00min. E: 4h 30min. (Amiloride $\Delta I_{sc}$ data extracted for preparation of Fig. 6) .

Amiloride 10μM final concentration was added to the apical surface at 30min. after Krebs- Ringer buffer wash and assembling in Ussing chambers, forskolin was added apically and basolaterally at 37min.

**Figure 8:**

Amiloride $\Delta I_{sc}$ response of HBE cells treated with 6- amidino- 2- napthyl 4- guanidinobenzoate metabolites. A: HBE cells cultured for 22d, 17d at ALI. Treated for 90 min with $10^{-6}$M- or $10^{-4}$M- pGBA (closed triangles), or $10^{-6}$M 6- amidino- 2- napthyl 4- guanidinobenzoate (closed circles) . Non- treatment controls (open circles) . B: HBE cells cultured for 34 & 35d, 28 & 29d at ALI. Treated for 90min with $10^{-4}$M- or $10^{-6}$M- AN (closed inverted triangles), mixture of $10^{-6}$M- metabolites pGBA & AN (closed diamonds) or $10^{-6}$M 6- amidino- 2- napthyl 4- guanidinobenzoate (closed circles) . Non- treatment controls (open circles) . Amiloride 10μM final concentration was added to the apical surface 30min. after Krebs- Ringer buffer wash and assembly in Ussing chambers. Controls and treatment groups analysed by one- way ANOVA, t- test used to compare control and 6- amidino- 2- napthyl 4- guanidinobenzoate treated groups.

**Figure 9:**

Nasal PD responses to perfusion with $10^{-5}$M- amiloride. Responses were measured in two periods before perfusion with $3.10^{-6}$M- 6- amidino- 2- napthyl 4- guanidinobenzoate; and in three periods after perfusion with $3.10^{-6}$M- 6- amidino- 2- napthyl 4- guanidinobenzoate.

**Figure 10:**

Incidence of mucus host-defence episodes during the perfusion schedule for study of nasal PD in each of four experiments. Responses were scored 1 to 3 based on the combination of symptoms and the magnitude of the response for each episode.

**Detailed Description of Invention**

The current inventors have found 6- amidino- 2- napthyl 4- guanidinobenzoate to be a potent long duration inhibitor of ENaC activity in airway epithelial cells. This and related compounds therefore have potential in the treatment of respiratory diseases characterised by mucostasis such as cystic fibrosis (CF) ; bronchiectasis; chronic bronchitis; chronic obstructive pulmonary disease (COPD) ; rhino- sinusitis and otitis media.

The preferred respiratory disease for treatment with such compounds is cystic fibrosis.

**Potency:**

The profile of activity of 6- amidino- 2- napthyl 4- guanidinobenzoate shows similarities to the profiles reported for the Kunitz- domain serine protease inhibitors BAY 39- 9437 and aprotinin (Bridges et al. 2001) . The current inventors have achieved a maximum down regulation of ENaC activity with 6- amidino- 2- napthyl 4- guanidinobenzoate in the range 50% to 70% of the inhibition achieved by application of high dose amiloride to human airway epithelial cell cultures. BAY 39- 9437 and aprotinin achieved efficacy in the same range (Bridges et al. 2001) compared with high dose amiloride. The half maximal inhibitory concentration for 6- amidino- 2- napthyl 4- guanidinobenzoate is in the range 30 to 100nM, which compares favourably to BAY 39- 9437 (reported as approximately 25nM) .

Unlike with amiloride, onset of down regulation of ENaC activity by serine protease inhibitors is not immediate. 6- amidino- 2- napthyl 4- guanidinobenzoate achieves half maximal inhibition by 5 min and maximum down regulation by 30 min. Again, this compares favourably with BAY 39- 9437 where an onset $t_{1/2}$ of 45 min was reported (Bridges et al 2001) .

**Duration of Action:**

An important aspect to the profile of 6- amidino- 2- napthyl 4- guanidinobenzoate is its long duration of action. Significant down regulation of ENaC is still observed 1h 45min after washout of the 6- amidino- 2- napthyl 4- guanidinobenzoate, which represents 78% of the maximal down regulation observed at 30min after washout.

Cells treated with 6- amidino- 2- napthyl 4- guanidinobenzoate and transferred to fresh culture for 3h 00min prior to assembly into Ussing chambers for short circuit current recording show, in the first 15 minutes of recording, a slope of rising baseline short circuit current that is over 20 times that recorded for untreated controls (Figures 7D and 7A) . The increased slope of the rising baselines in these cells suggests that activation of ENaC is triggered at the start of recording by the actions of assembling the cell cultures into the Ussing chambers and initiating voltage clamping. This

interpretation of the recordings suggests that the duration of down regulation of ENaC after washout of 6- amidino- 2- napthyl 4- guanidinobenzoate is considerably underestimated for undisturbed cell cultures.

The long duration of down regulation achieved by 6- amidino- 2- napthyl 4- guanidinobenzoate again compares favourably with BAY 39- 9437 where a half- life of 15 min. was reported (Bridges et al. 2001) .

The pharmacological profile of 6- amidino- 2- napthyl 4- guanidinobenzoate as a potent long duration down regulator of epithelial sodium channel activity in human airway epithelial cells is not predicted by previous studies.

The term "respiratory diseases" includes but is not limited to conditions of the lung, airways (bronchioles, bronchi), and upper respiratory tract (nose, para-nasal sinuses, Eustachian tube and middle ear) characterised by impaired mucociliary clearance, possibly resulting in mucostasis. Such conditions include but are not limited to: cystic fibrosis (CF); bronchiectasis; chronic bronchitis; chronic obstructive pulmonary disease (COPD); rhino-sinusitis; otitis media.

The term "impermeant" refers to solutes that are unable to pass through a cell membrane.

The current invention will be further understood by way of the following examples.

## General methods

Examples 1 to 6 use the same set of material and methods, which are described below.

### Cells

Human bronchial epithelial (HBE) cells were purchased from Cambrex Bioscience Ltd, UK., and were stored frozen in liquid nitrogen until required for use.

### Cell culture

HBE cells were amplified by growing in secondary culture according to the suppliers' instructions. Cells were seeded at densities of between 3,000 to 4,000/$cm^2$ in plastic T-25 culture flasks in bronchial epithelial cell growth medium (BEGM) prepared from bronchial epithelial basal medium (BEBM, Cambrex, UK) supplemented with bovine pituitary extract, hydrocortisone, human epidermal growth factor, epinephrine, transferrin, insulin, retinoic acid, triiodothyronine, gentamicin, and amphotericin B. Cultures were maintained at 37°C in 5% $CO_2$ in a humidified incubator. Medium was changed every 1 to 3 days until cells were approximately 90% confluent. Cells were then passaged with trypsin EDTA according to the suppliers' instructions, and either used for further cell cultures, or cryopreserved in liquid nitrogen in BEGM containing 10% foetal bovine serum and 10% DMSO.

HBE cells were prepared for electrophysiology studies by growing as tertiary cultures on Snapwell (Corning) 0.4μ clear polyester membrane inserts 1.2 $cm^2$ coated with collagen IV (Sigma) . Cells were seeded at approximately $10^5$/$cm^2$ in 0.5ml 1: 1 DMEM (Cambrex, UK) BEGM containing bovine pituitary extract (50μg/ml), hydrocortisone (0.5μg/ml), human epidermal growth factor (0.5ng/ml), epinephrine (0.5μg/ml), transferrin (10μg/ml), insulin (5μg/ml), retinoic acid (50nM), and gentamicin (50μg/ml) (Danahay medium; Danahay et al. 2002) . Snapwell inserts were mounted in 6- well plates with 3ml Danahay medium in the wells, and grown at 37°C in 5% $CO_2$ in a humidified incubator. Medium was changed every 1 to 3 days. An apical air- liquid interface (ALI) was established as the cells achieved confluence (3 to 6 days) by removal of medium from the centre of the insert. Confluent differentiated bronchial epithelial cells were used for electrophysiology studies at between 15 and 29 days after establishing ALI.

### Drug treatment protocols and measurement of short circuit current ($I_{sc}$)

Differentiated HBE cells grown to confluence at an air-liquid interface for 15 to 29 days were used to study substances for ability to achieve long duration down regulation of the epithelial amiloride-sensitive sodium channel (ENaC).

Step 1: Treatment with substance. For each group of cells treated with a substance, 30μl of 100 times final concentration of substance was added to the 3ml basolateral culture medium, mixed and 150μl of the resultant basolateral fluid was transferred to the apical surface for 5min. For non-treatment control groups of basolateral fluid with no added substance was transferred to the apical surface. After 5min. equilibration the excess apical fluid was then transferred back to the basolateral side of the culture well.

Step 2: Variable period of incubation with the substance. Dependent on the individual experiment the cells were returned to the 37°C incubator for periods of 0min. to 120min.

Step 3: Period of substance washout with fresh culture medium. For experiments to study the duration of a substance response the HBE cells on Snapwell inserts were washed with fresh culture medium (no added substance) at 37°C and placed in fresh 6-well plates containing 3ml fresh culture medium (no added substance) on the basolateral

side. Cells were returned to the 37°C incubator for periods of 0 minutes to 120 minutes. Step 3 was omitted in studies where the duration of the substance response was not investigated.

Step 4: Wash with Krebs- Ringer buffer and assembly in Ussing chambers. The Snapwell insert cultures of HBE cells were removed from the 6- well plates and washed with excess Krebs- Ringer buffer (120mM- NaCl, 25mM- $NaHCO_3$, 3.3mM- $KH_2PO_4$, 0.8mM- $K_2HPO_4$, 1.2mM- $CaCl_2$, 1.2mM- $MgCl_2$, 10mM- glucose) pH7.4 at 37°C. The inserts were then mounted in Ussing chambers (Snapwell diffusion chambers, Costar UK Ltd.) . Each side of the chamber was filled with 5ml Krebs- Ringer buffer and gassed continuously with 95%$O_2$/ 5%$CO_2$ at 37°C.

Step 5: Short circuit current ($I_{sc}$) measurement. Cells were voltage clamped to 0mV using a WPI EVC 4000 voltage clamp (World Precision Instruments, UK). Silver/silver chloride electrodes (Costar Ltd., UK) were used to monitor $I_{sc}$, which was recorded continuously throughout each experiment using AcqKnowledge III for MP100WSW data acquisition software (Biopac, Linton Instruments, UK). Transepithelial resistance ($R_T$) was determined every minute by delivering a 5mV pulse for 3 seconds and recording the change in $I_{sc}$, over the 3 second period ($\Delta I_{sc}$). $R_T$ was was calculated using the Ohm's law relationship $R_T = [V(5mV) / \Delta I_{sc}]$.

At 30 min. after assembling the cultured HBE cells in Ussing chambers, 5μl of $10^{-2}$ M-amiloride (final chamber concentration 10 μM) was added to the apical surface. ENaC short circuit current activity was determined for each culture by measuring the difference between $I_{sc}$ immediately prior to addition of amiloride and at 3min. following addition of the agent (Amiloride $\Delta I_{sc}$).

At 37 min. 5μl of $10^{-2}$ M-forskolin (final chamber concentration 10 μM) was added to both the apical and basolateral surfaces. Forskolin stimulated a cAMP-dependent chloride channel current by activation of the cystic fibrosis transmembrane regulator (CFTR) in the epithelial cells. The magnitude of the CFTR $I_{sc}$ response to forskolin was determined for each culture by measuring the difference between $I_{sc}$ immediately prior to addition of forskolin and at plateau at approximately 6min. following addition of the agent (Forskolin $\Delta I_{sc}$).

**Data analysis**

An unpaired t-test was used to analyse experiments where two data groups were compared; statistical significant difference level $p \leq 0.05$.

One- way analysis of variance (ANOVA) was used to analyse studies where more than two data groups were compared. When statistical significance was achieved $p \leq 0.05$ in ANOVA, Tukey's multiple comparison test was used to compare between individual groups.

**Example 1: Short duration inhibition of ENaC activity by amiloride**

Figure 1 illustrates the short circuit current response of HBE cells to 10μM-amiloride added to the apical side of cells mounted in an Ussing chamber. Inhibition of ENaC activity occurred rapidly. Typically short circuit current was reduced to a new plateau level within 2 min of application of amiloride to the apical surface.

When amiloride was removed from contact with the cell preparation by washout with fresh Krebs-Ringer buffer of both the apical and basolateral sides of the Ussing chamber, short circuit current increased rapidly to plateau within 2 to 5 min. (Figure 1) close to the pre-amiloride current level.

**Example 2: Pre-treatment of HBE cells for 90min with 1μM 6-amidino-2-napthyl 4-guanidinobenzoate**

HBE cells cultured for 24 & 25 days, and 19 & 20 days at ALI were pre- treated for 90min with 1μM 6- amidino- 2- napthyl 4- guanidinobenzoate in culture medium. Control cultures were treated according to the same protocol, but without 6- amidino- 2- napthyl 4- guanidinobenzoate in the culture medium. After 90min the substance treated cultures were washed free of 6- amidino- 2- napthyl 4- guanidinobenzoate with Krebs- Ringer buffer, and then assembled in Ussing chambers with 5ml buffer each side of the chamber. Again control cultures were treated according to the same protocol.

At 30 min following assembly of the Ussing chambers 10μM- amiloride was added to the apical surface of cells. The resulting fall in short circuit current (amiloride $\Delta I_{sc}$) in response to application of amiloride (approximately 10min. after start of recording) was greater in control cultures (Figures 2A & 2C) compared to cultures pre- treated with 6- amidino- 2- napthyl 4- guanidinobenzoate (Figures 2B & 2D) . Figure 3 illustrates two studies in which amiloride $\Delta I_{sc}$ was significantly ($p<0.001$) reduced, by 60% and 57%, in 1μM 6- amidino- 2- napthyl 4- guanidinobenzoate, 90 min pre-treated cultures (Figure 3A) compared to control cultures. The reduction in amiloride $\Delta I_{sc}$ indicates reduced ENaC activity as the result of 6- amidino- 2- napthyl 4- guanidinobenzoate treatment. It should also be noted that the reduced ENaC activity was seen 30 min after 6- amidino- 2- napthyl 4- guanidinobenzoate had been washed away from the cultured cells. This demonstrates that 6- amidino- 2- napthyl 4- guanidinobenzoate exerts a long duration down regulatory effect on the epithelial sodium channel in HBE cells. Amiloride was not washed away from the cultures after plateau (Figure

2), and at 37 min following assembly of the Ussing chambers 10μM- forskolin was added to both the apical and basolateral sides of the chambers. Short circuit current was observed to increase (forskolin $\Delta I_{sc}$) in all cultures (Fig 2) . The increase in short circuit current in response to forskolin is consistent with activation of CFTR anion conductance in HBE cells. Figure 3 illustrates that in contrast to the amiloride $\Delta I_{sc}$ , 1μM 6- amidino- 2- napthyl 4- guanidinobenzoate, 90 min pre-treatment had no significant effect on forskolin $\Delta I_{sc}$ compared to control (Figure 3B) .

**Example 3: Dose-response to 6-amidino-2-napthyl 4-guanidinobenzoate treatment**

HBE cells cultured for 18 & 19 days, and 15 & 16 days at ALI were pre- treated for 90min with 6- amidino- 2- napthyl 4- guanidinobenzoate over the dose range 30nM to 3μM in culture medium. Amiloride $\Delta I_{sc}$ for 6- amidino- 2- napthyl 4- guanidinobenzoate treated cultures was reduced compared to control in a dose related manner (Figure 4A) . Pre- treatment with 3μM- 6- amidino- 2- napthyl 4- guanidinobenzoate resulted in 54% reduction in amiloride $\Delta I_{sc}$ ($p<0.001$) ; 1μM in 52% reduction ($p<0.01$) ; 0.3μM in 42% reduction ($p<0.05$) ; 0.1μM in 40% reduction ($p<0.05$) ; and 30nM in 13% reduction (ns) (Figure 4A) . Again the reduced amiloride $\Delta I_{sc}$ response was seen 30min after 6- amidino- 2- napthyl 4- guanidinobenzoate had been washed away from the cultured cells.

Figure 4B illustrates that 6- amidino- 2- napthyl 4- guanidinobenzoate pre- treatment (90 min) across the dose range 30nM to 3μM was without effect on forskolin $\Delta I_{sc}$ compared to control.

**Example 4: Time of onset study** of **down regulation of amiloride $\Delta I_{sc}$**

HBE cells cultured for 22 days, 19days at ALI, were studied to assess down regulation of amiloride $\Delta I_{sc}$ in response to exposure to 1μM 6- amidino- 2- napthyl 4- guanidinobenzoate in culture medium for pre- treatment times over the range 5min to 60min. Again after pre- treatment the cultures were washed free of 6- amidino- 2- napthyl 4- guanidinobenzoate with Krebs- Ringer buffer before mounting in Ussing chambers. Figure 5 shows the time dependent relationship in the amiloride $\Delta I_{sc}$ response. Significantly reduced amiloride $\Delta I_{sc}$ response was observed at all time points studied; 31% reduction vs control ($p<0.001$) at 5 min pre- treatment, 48% ($p<0.001$) at 10 min, 55% ($p<0.001$) at 20 min, 61% ($p<0.001$) at 30 min, and 58% ($p<0.001$) at 60 min.

No significant difference between treatment and control was observed for the forskolin $AI_{sc}$ response over the pre-treatment time range studied.

**Example 5: Duration of the amiloride $\Delta I_{sc}$ response**

The effect of time were further studied by evaluating the effect of a period of washout on amiloride $\Delta I_{sc}$ responses. HBE cells cultured for 26 days, 23days at ALI, were pre- treated with 1μM 6- amidino- 2- napthyl 4- guanidinobenzoate for 60 min. Cells were then washed free of 6- amidino- 2- napthyl 4- guanidinobenzoate with fresh culture medium and returned to the incubator for between 0min and 4h 30min. Cells were then washed as before with Krebs- Ringer buffer before mounting in Ussing chambers.

Figure 6 shows that culture medium washout periods of 0min and 1h 15min resulted in significantly reduced amiloride $\Delta I_{sc}$ compared to control; 68% reduction vs control ($p<0.001$) for 0 min culture medium washout, 53% ($p<0.001$) for 1h 15min. It should again be noted that the 30 min. time interval between wash with Krebs- Ringer buffer and addition of amiloride means that the total time elapsed after removal of 6- amidino- 2- napthyl 4- guanidinobenzoate was 30min and 1h 45min respectively for these two groups.

By contrast the amiloride $\Delta I_{sc}$ for the 3h 00min culture medium washout period was elevated (+34%, $p<0.001$) compared to control (Figure 6) . However inspection of the short circuit current recordings (Figure 7) illustrates a further complexity in interpretation of the duration of response data. In Figure 7A it can be seen that the baseline $I_{sc}$ for the five control cultures is relatively stable over the initial recording period 0 to 12min prior to addition of amiloride, average baseline slope +0.04μAmp/min. This contrasts particularly with the short circuit current recordings for the equivalent period in Fig 7D; 6- amidino- 2- napthyl 4- guanidinobenzoate pre- treated cultures followed by 3h 00min washout with culture medium. Here there is a rising baseline $I_{sc}$ for the initial period in all five cultures, average baseline slope +0.93μAmp/min., but this is followed by a stable plateau after inhibition of ENaC by addition of amiloride. This pattern suggests that transfer of the cells from culture medium to Krebs- Ringer buffer and voltage clamping the cultures at 0mV may have triggered activation of sodium channels at this point in the 3h washout cells.

Figure 6 further shows that for the 4h 30min culture medium washout group amiloride $\Delta I_{sc}$ was not significantly different to control. However the rising baseline in the initial period of short circuit current recording (Fig 7E), average baseline slope +0.60μAmp/min., again suggests triggering of activation of sodium channels.

**Example 6: 6-amidino-2-napthyl 4-guanidinobenzoate metabolites do not down regulate amiloride $\Delta I_{sc}$**

6- amidino- 2- napthyl 4- guanidinobenzoate is known to be metabolised in plasma to compounds p- guanidinobenzoic acid (pGBA) and 6- amidino- 2- naphthol (AN) . HBE cells cultured for 22 days, 17 days at ALI were pre-treated for 90 min with 1$\mu$M or 100$\mu$M- pGBA, or 1$\mu$M 6- amidino- 2- napthyl 4- guanidinobenzoate. Amiloride $\Delta I_{sc}$ for the pGBA treated groups was not significantly different from control (Figure 8A) . Cultures pre- treated with 1$\mu$M 6-amidino- 2- napthyl 4- guanidinobenzoate for 90 min showed amiloride $\Delta I_{sc}$ reduced by 57% ($p<0.0001$) compared to control.

HBE cells cultured for 34 & 35 days, 28 & 29 days at ALI were pre- treated for 90 min with 1$\mu$M or 100$\mu$M- AN, or 1$\mu$M- pGBA plus 1$\mu$M- AN, or 1$\mu$M 6- amidino- 2- napthyl 4- guanidinobenzoate. Amiloride $\Delta I_{sc}$ for the AN treated groups and for the combined pGBA and AN group was not significantly different from control (Figure 8B) . Cultures pre-treated with 1$\mu$M 6- amidino- 2- napthyl 4- guanidinobenzoate for 90 min showed amiloride $\Delta I_{sc}$ reduced by 45% ($p<0.0001$) compared to control.

Various aspects of the present invention have been exemplified in the preceding part of the specification. These formulations and methods discussed are intended for exemplification purposes only and are not intended to limit the scope of the accompanying claims.

**Example 7: Perfusion of human nasal epithelium with 6-amidino-2-napthyl 4-guanidinobenzoate**

Preliminary experiments to monitor effects of perfusion of 6- amidino- 2- napthyl 4- guanidinobenzoate on the nasal epithelium on the amiloride- sensitive (sodium- dependent) component of nasal PD in a volunteer normal human subject have been carried out. The following methods were used:

**Methods**

The method of measurement of nasal PD was an adaptation of that described by Standaert et al. (2004).

**Solutions**

Ringer/ HEPES buffer solution was prepared containing 148mM- NaCl., 4.05mM- KCl, 1.2mM- $CaCl_2$, 1.2mM- $MgCl_2$, 2.4mM- $K_2HPO_4$, 0.4mM- $KH_2PO_4$, 10mM- HEPES, and adjusted to pH 7.4.

Low chloride Ringer/HEPES buffer solution was prepared by replacing NaCl with 148mM-Na gluconate in the Ringer solution components.

**Electrodes and equipment**

Agar electrodes were prepared with 3.7% agarose dissolved in Ringer/HEPES buffer pH 7.4. The reference electrode was placed and taped in contact with a small area on the forearm where the epidermis had been removed by gentle abrasion. A dual lumen nasal recording electrode was prepared using a 1m length of soft plastic tubing 2.4mm diameter, into which 1mm diameter polythene tubing was inserted through the wall and along the lumen for 15 cm from one end. The 2.4mm diameter tubing was filled with 3.7% agarose dissolved in Ringer/HEPES buffer pH 7.4. The 1mm diameter tubing was used for perfusion of buffer solutions and drugs to the nasal epithelium. The ends of the agar electrodes were each placed in contact with a calomel half-cell in 3M-KCl solution. Voltages were measured by connecting the calomel electrodes to a high impedance voltmeter (Keithley 617 programmable electrometer). Output from the voltmeter was recorded by connection to a Biopac MP100 data aquisition unit and PC running Acqknowledge III data acquisition software.

**Nasal PD measurement**

Nasal PD was recorded via the recording electrode placed in contact with the nasal epithelium approximately 4cm inside the:nostril. The nasal epithelium was perfused according to the following schedule and nasal PD was recorded continuously.

1. Ringer/HEPES buffer was perfused at 0.5ml/min for 5min, and starting baseline PD was recorded.
2. Perfusion was then switched to low chloride Ringer/HEPES buffer pH 7.4 for 30min during which time initial hyperpolarisation of the epithelium occurred.
3. The nasal epithelium was then perfused with $10^{-5}$M- amiloride in low chloride Ringer/ HEPES buffer pH 7.4 for 10min. Measurement of the amiloride- sensitive (sodium- dependent) component of nasal PD was then made by

measuring the difference between the average PD recorded over the 2min before perfusion with amiloride and the average PD recorded in the period 4 to 6min after perfusion with amiloride.

4. Amiloride was washed away from the nasal epithelium by perfusion with low chloride Ringer/HEPES buffer pH 7.4 for 30min.
5. Steps 3 and 4 were then repeated twice more.
6. The nasal epithelium was then perfused with 3 μM 6- amidino- 2- napthyl 4- guanidinobenzoate in low chloride Ringer/ HEPES buffer pH 7.4 for 60min.
7. Steps 3 and 4 were then repeated a further twice, followed by either a period of administration of amiloride (3 experiments), or a final repetition of both steps 3 and 4 (1 experiment).

**Results**

**Nasal PD**

The nasal PD protocol was performed on 4 separate days, a minimum of 2 days apart. In the initial period of perfusion with Ringer/HEPES buffer the mean nasal PD was -6.1mV (range 0 to -10.4mV). Perfusion with low chloride Ringer/HEPES buffer caused gradual hyperpolarization of the nasal epithelium. At the end of the second period of perfusion with low chloride Ringer/HEPES buffer the mean nasal PD was -13.1mV (range -6.3 to -20.3mV).

Over the 5h time course of each experiment nasal PD drifted and oscillated. However, nasal PD was sufficiently stable over periods of 10min to allow meaningful measurements of responses to perfusion of amiloride to be made. Characteristically perfusion of the nasal epithelium with $10^{-5}$M- amiloride caused partial depolarisation of the epithelium that was observable within 2min of initiation of perfusion of the drug, and plateaued within 4 to 6min. Changes in nasal PD caused by perfusion of amiloride were calculated as the difference between the mean PD recorded in the 2min period before perfusion with amiloride and the mean PD recorded in the plateau period 4 to 6min after start of perfusion with amiloride. Within each experiment the change in nasal PD at each period of application of amiloride was expressed as a percentage of the maximum change observed in the experiment. Figure 9 shows for 4 experiments the average percent of maximum PD change in response to $10^{-5}$M- amiloride in the periods 1) 70min before perfusion of $3.10^{6}$M- 6-amidino- 2- napthyl 4- guanidinobenzoate (94%) ; 2) 30 min before (80%) ; 3) immediately following perfusion of $3.10^{-6}$M- 6- amidino- 2- napthyl 4- guanidinobenzoate for 60min (49%) ; 4) 40min after (29%) ; 5) 80min after (55%) .

**Mucus host-defence responses**

Perfusion of the nasal epithelium with low chloride Ringer/ HEPES buffer imposes a chemical gradient for chloride secretion, and therefore also provides a drive for fluid secretion. Periods of perfusion with $10^{-5}$M- amiloride and with $3.10^{-6}$M- 6- amidino- 2- napthyl 4- guanidinobenzoate stimulated episodes of mucus host- defence responses characterised in any episode by recording one or more of the following features:

- Post nasal sensation; drip and/or catarrh; taste
- Mucus gel secretion exuding from nostril; perfused nostril and/or contralateral nostril
- Sneeze(s)
- Itch in nostril; unilateral or bilateral
- Eye watering
- Blockage of the nostril; unilateral or bilateral

Mucus host-defence episodes were scored 1 to 3 on the basis of the number of features and the magnitude of the responses observed. Figure 10 reports the score of the episodes recorded, and indicates the time of their occurrence in the perfusion protocol.

**Conclusions**

- Perfusion of the nasal epithelium with $3.10^{-6}$M- 6- amidino- 2- napthyl 4- guanidinobenzoate in low chloride Ringer/ HEPES buffer pH 7.4 for 60min reduced the amiloride- sensitive (sodium- dependent) component of nasal PD for up to 80min following perfusion of 6- amidino- 2- napthyl 4- guanidinobenzoate.
- Periods of perfusion with either $10^{-5}$M- amiloride or with $3.10^{-6}$M- 6- amidino- 2- napthyl 4- guanidinobenzoate in low chloride Ringer/ HEPES buffer stimulated episodes of mucus host- defence responses where a component of the response was consistent with ciliary clearance of mucus gel in the sinuses and on the nasal epithelium.

- Observation of a mucus host- defence response to 6- amidino- 2- napthyl 4- guanidinobenzoate is novel.
- Highest scores for episodes of mucus host- defence response were recorded during periods of perfusion of $3.10^6$M- 6- amidino- 2- napthyl 4- guanidinobenzoate in low chloride Ringer/ HEPES buffer.

**References**

Bridges RJ, Newton BB, Pilewski JM, Devor DC, Poll CT. & Hall RL.Na+ transport in normal and CF human bronchial epithelial cells is inhibited by BAY 39-9437.Am J Physiol Lung Cell Mol Physiol. 2001; 281: L16-23.

Danahay H, Atherton H, Jones G, Bridges RJ. & Poll CT. Interleukin-13 induces a hypersecretory ion transport phenotype in human bronchial epithelial cells.Am J Physiol Lung Cell Mol Physiol. 2002; 282: L226-236.

Hitomi Y, Ikari N, Fujii SInhibitory effect of a new synthetic protease inhibitor (FUT-175) on the coagulation system.Haemostasis. 1985;15: 164-8

Iwaki M, Oda M, Ozeki M, Ino Y, Suzuki K, Koshiyama Y, Motoyoshi A, Ogihara M, Suzuki S, Fujita M, et al. [Pharmacological studies of FUT-175, nafamstat mesilate. III. Anti-inflammatory activities of FUT-175].Nippon Yakurigaku Zasshi. 1984;84: 373-84.

Iwashita K, Kitamura K, Narikiyo T, Adachi M, Shiraishi N, Miyoshi T, Nagano J, Tuyen DG, Nonoguchi H. & Tomita K. Inhibition of prostasin secretion by serine protease inhibitors in the kidney.J Am Soc Nephrol. 2003; 14: 11-16.

Muto, S, Imai M. & Asano Y.Effect of nafamostat mesilate on Na+ and K+ transport properties in the rabbit cortical collecting duct. Br. J. Pharmacol. 1993; 109: 673-678.

Muto S, Imai M. & Asano Y.Mechanisms of the hyperkalaemia caused by nafamostat mesilate: effects of its two metabolites on Na+ and K+ transport properties in the rabbit cortical collecting duct. Br.J.Pharmacol. 1994; 111: 173-178.

Narikiyo T, Kitamura K, Adachi M, Miyoshi T, Iwashita K, Shiraishi N, Nonoguchi H, Chen LM, Chai KX, Chao J. & Tomita K.Regulation of prostasin by aldosterone in the kidney. J Clin Invest. 2002; 109: 401-408.

Rossier BC.The epithelial sodium channel: activation by membrane-bound serine proteases.Proc Am Thorac Soc. 2004; 1: 4-9.

Standaert TA, Boitano L, Emerson J, Milgram LJ, Konstan MW, Hunter J, Berclaz PY, Brass L, Zeitlin PL, Hammond K, Davies Z, Foy C, Noone PG, Knowles MR.

Standardized procedure for measurement of nasal potential difference: an outcome measure in multicenter cystic fibrosis clinical trials.Pediatr Pulmonol. 2004; 37 (5) : 385- 92.

Tong Z, Illek B, Bhagwandin VJ, Verghese GM. & Caughey GH. Prostasin, a membrane-anchored serine peptidase, regulates sodium currents in JME/CF15 cells, a cystic fibrosis airway epithelial cell line.Am J Physiol Lung Cell Mol Physiol. 2004; 287: L928-935.

Vallet V, Chraibi A, Gaeggeler HP, Horisberger JD. & Rossier BC.An epithelial serine protease activates the amiloride-sensitive sodium channel.Nature. 1997; 389: 607-610.


**Claims**

**1.** Use of an amidino compound of the general formula:

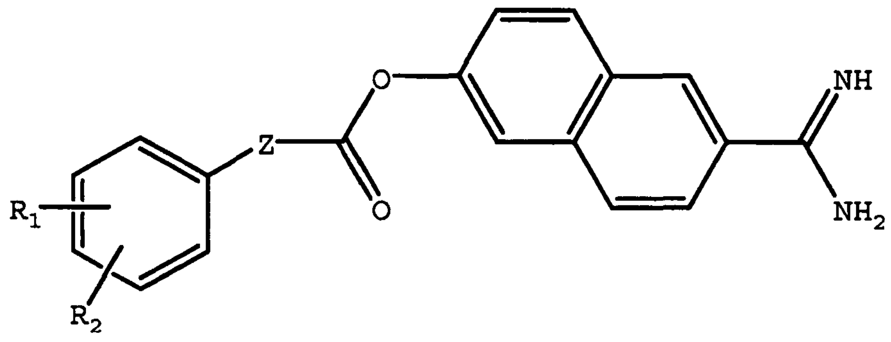

or a pharmaceutically acceptable salt thereof, in the manufacture of a medicament for the treatment of respiratory diseases **characterised by** poor mucociliary clearance or mucostasis wherein;
Z represents-- $(CH_2)_a$-,

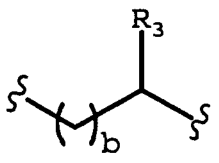 , 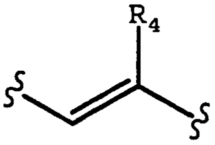 or 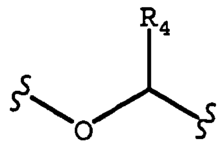

wherein a is 0, 1, 2 or 3, b is 0, 1 or 2, $R_3$ is a straight or branched chain alkyl group of 1 to 4 carbon atoms or a cycloalkyl group of 3 to 6 carbon atoms, $R_4$ is a hydrogen atom or a straight or branched chain alkyl group of 1 to 4 carbon atoms;
$R_1$ and $R_2$, may be the same or different and represent each a hydrogen atom, a straight or branched chain alkyl group of 1 to 4 carbon atoms, -O- $R_5$, -S- $R_5$, -$COOR_5$, -$COR_6$, -O- $COR_7$, -$NHCOR_7$ ,

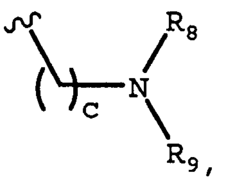 , 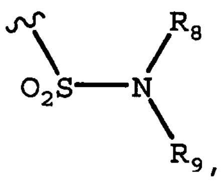 , 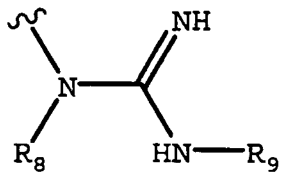

$NO_2$, CN, halogen, $CF_3$, methylenedioxy, or

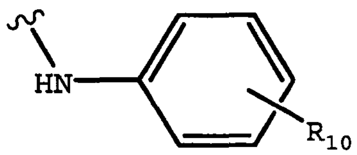

wherein c is 0, 1 or 2; $R_5$ is a hydrogen atom, linear or branched chain alkyl group of 1 to 4 carbon atoms, or benzyl group; $R_6$ is a hydrogen atom or straight or branched chain alkyl group of 1 to 4 carbon atoms; $R_7$ is a straight or branched chain alkyl group of 1 to 4 carbon atoms; $R_8$ and $R_9$, which may be the same or different, are each a hydrogen atom, straight or branched chain alkyl group of 1 to 4 carbon atoms, or amino radical protecting group; and $R_{10}$ is a hydrogen atom, methyl or $CF_3$, wherein the medicament is an aqueous solution with a chloride concentration of less than 10 mM.

2. The use according to claim 1, wherein the amidino compound is in the form of a pharmaceutically acceptable salt or ester.

3. The use according to any preceding claim, wherein the amidino compound is 6- amidino- 2- napthyl 4- guanidinobenzoate.

4. The use according to claim 3, wherein the 6- amidino- 2- napthyl 4- guanidinobenzoate is in the form of 6- amidino-2- napthyl 4- guanidinobenzoate dihydrochloride or 6- amidino- 2- napthyl 4- guanidinobenzoate mesylate.

5. The use of any preceding claim, wherein the medicament further comprises one or more pharmaceutically acceptable carriers or diluents.

6. The use according to claim 5, wherein the medicament further comprises a pharmaceutically acceptable impermeant, monovalent anion in the range of from 100mM to 160mM, preferably in the range of from 140mM to 150mM.

7. The use according to claim 5, wherein the pharmaceutically acceptable impermeant, monovalent anion is gluconate.

8. The use of any preceding claim wherein the amidino compound is the sole active ingredient.

9. The use of any one of claims 1 to 7 wherein the medicament also comprises one or more other active agents selected from the group of antibiotics, vaccines, decongestants (nasal or bronchial), mucolytic agents, rhDNase, non-steroidal antiinflamatory agents (NSAIDs), steroids, antiviral agents, elastase inhibitors, exofacial sodium channel blocking agents, gene therapy agents, chloride channel activators, mannitol, purinergic receptor agonists and bronchodilators.

10. The use of claim 9 wherein the medicament comprises only one additional active agent.

11. The use of any preceding claim, wherein the respiratory disease is selected from the group comprising cystic fibrosis (CF); bronchiectasis; chronic bronchitis; chronic obstructive pulmonary disease (COPD) and rhino-sinusitis.

12. The use according to any preceding claim, wherein the respiratory disease is cystic fibrosis.

13. The use according to any preceding claim, wherein the medicament is formulated for administration to the respiratory system by the pulmonary route.

14. The use according to claim 13, wherein the medicament is formulated for administration by any one of intratracheal instillation, intratracheal delivery of liposomes, insufflation, nebulization and aerosol inhalation.

15. The use according to claim 14, wherein the medicament is formulated for administration by aerosol inhalation, together with a propellant selected from the group comprising hydrofluroalkanes, chlorofluorocarbons, propane, nitrogen, or a mixture thereof.

16. The use of any one of claims 1 to 12 wherein the medicament is formulated for the treatment of the nasal epithelium, para-nasal sinuses, said medicaments being in the form of solutions for delivery as drops, by pipette or by syringe to the nasal epithelium, para-nasal sinuses, for the irrigation of the para-nasal sinuses.

17. A medicament for use in the treatment of respiratory diseases **characterised by** poor mucociliary clearance or mucostasis as defined in any of claims 1 to 16.

18. An inhalation device loaded with the medicament as defined in claim 17.

19. The inhalation device of claim 18, wherein the device is a metered dose inhaler, jet nebulizer or ultrasonic nebulizer.

**Patentansprüche**

1. Verwendung einer Amidinoverbindung der allgemeinen Formel

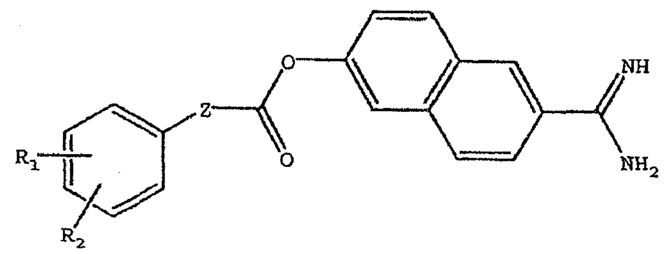

oder eines pharmazeutisch verträglichen Salzes davon bei der Herstellung eines Medikaments zur Behandlung von Atemwegserkrankungen, die **gekennzeichnet sind durch** schlechte Mukoziliar-Clearance oder Mukostase, worin Z $-(CH_2)_a-$,

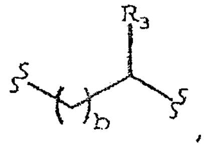
,
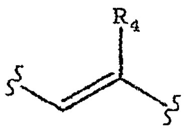
oder
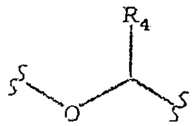

bedeutet, worin a 0, 1, 2 oder 3 ist, b 0, 1 oder 2 ist, $R_3$ eine geradkettige oder verzweigtkettige Alkylgruppe mit 1 bis 4 Kohlenstoffatomen oder eine Cycloalkylgruppe mit 3 bis 6 Kohlenstoffatomen ist, $R_4$ ein Wasserstoffatom oder eine geradkettige oder verzweigtkettige Alkylgruppe mit 1 bis 4 Kohlenstoffatomen ist;
$R_1$ und $R_2$ gleich oder verschieden sein können und jeweils ein Wasserstoffatom, eine geradkettige oder verzweigtkettige Alkylgruppe mit 1 bis 4 Kohlenstoffatomen, $-O-R_5$, $-S-R_5$, $-COOR_5$, $-COR_6$, $-O-COR_7$, $-NHCOR_7$,

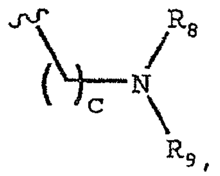
,
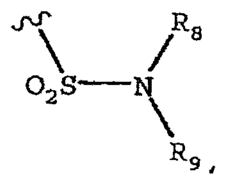
,
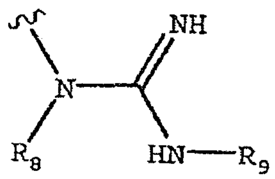
,

$NO_2$, CN, Halogen, $CF_3$, Methylendioxy oder

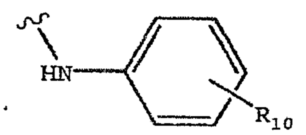

bedeuten,
worin c 0, 1 oder 2 ist; $R_5$ ein Wasserstoffatom, eine lineare oder verzweigtkettige Alkylgruppe mit 1 bis 4 Kohlenstoffatomen oder eine Benzylgruppe ist; $R_6$ ein Wasserstoffatom oder eine geradkettige oder verzweigtkettige Alkylgruppe mit 1 bis 4 Kohlenstoffatomen ist; $R_7$ eine geradkettige oder verzweigtkettige Alkylgruppe mit 1 bis 4 Kohlenstoffatomen ist; $R_8$ und $R_9$, die gleich oder verschieden sein können, jeweils ein Wasserstoffatom, eine geradkettige oder verzweigtkettige Alkylgruppe mit 1 bis 4 Kohlenstoffatomen oder eine Aminorest-Schutzgruppe sind; $R_{10}$ ein Wasserstoffatom, Methyl oder $CF_3$ ist, worin das Medikament eine wässrige Lösung mit einer Chloridkonzentration von weniger als 10 mM ist.

**2.** Verwendung nach Anspruch 1, worin die Amidinoverbindung in der Form eines pharmazeutisch verträglichen Salzes oder Esters ist.

3. Verwendung nach einem der vorhergehenden Ansprüche, worin die Amidinoverbindung 6- Amidino- 2- naphthyl- 4- guanidinobenzoat ist.

4. Verwendung nach Anspruch 3, worin das 6- Amidino- 2- naphthyl- 4- guanidinobenzoat in der Form von 6- Amidino- 2- naphthyl- 4- guanidinobenzoatdihydrochlorid oder 6- Amidino- 2- naphthyl- 4- guanidinobenzoatmesylat ist.

5. Verwendung nach einem der vorhergehenden Ansprüche, worin das Medikament weiterhin einen oder mehrere pharmazeutisch verträgliche Träger oder ein oder mehrere pharmazeutisch verträgliche Verdünnungsmittel enthält.

6. Verwendung nach Anspruch 5, worin das Medikament weiterhin ein pharmazeutisch verträgliches nichtpermeierendes monovalentes Anion im Bereich von 100 mM bis 160 mM, vorzugsweise im Bereich von 140 mM bis 150 mM umfasst.

7. Verwendung nach Anspruch 5, worin das pharmazeutisch verträgliche nichtpermeierende monovalente Anion Gluconat ist.

8. Verwendung nach einem der vorhergehenden Ansprüche, worin die Amidinoverbindung der einzige Wirkstoff ist.

9. Verwendung nach einem der Ansprüche 1 bis 7, worin das Medikament auch ein oder mehrere andere Wirkstoffe enthält, ausgewählt aus der Gruppe von Antibiotika, Impfstoffen, Decongestiva (nasal oder bronchial), mukolytischen Mitteln, rhDNase, nichtsteroidalen entzündungshemmenden Mitteln (NSAIDs), Steroiden, antiviralen Mitteln, Elastaseinhibitoren, exofaziale Natriumkanalblockierungsmitteln, Gentherapiemitteln, Chloridkanalaktivatoren, Mannitol, purinerger Rezeptor-Agonisten und Bronchodilatoren.

10. Verwendung nach Anspruch 9, worin das Medikament nur einen zusätzlichen Wirkstoff enthält.

11. Verwendung nach einem der vorhergehenden Ansprüche, worin die Atemwegserkrankung ausgewählt ist aus der Gruppe, umfassend zystische Fibrose (ZF); Bronchiektase; chronische Bronchitis; chronische obstruktive Lungenkrankheit (COPD) und Rhinosinusitis.

12. Verwendung nach einem der vorhergehenden Ansprüche, worin die Atemwegserkrankung zystische Fibrose ist.

13. Verwendung nach einem der vorhergehenden Ansprüche, worin das Medikament formuliert wird zur Verabreichung an das Atemwegssystem über den Lungenweg.

14. Verwendung nach Anspruch 13, worin das Medikament formuliert wird zur Verabreichung durch ein beliebiges aus intratrachealer Instillation, intratrachealer Zulieferung von Liposomen, Insufflation, Vernebelung und Aerosolinhalation.

15. Verwendung nach Anspruch 14, worin das Medikament formuliert wird zur Verabreichung durch Aerosolinhalation, zusammen mit einem Treibmittel, ausgewählt aus der Gruppe, umfassend Hydrofluoralkane, Chlorfluorkohlenstoffe, Propan, Stickstoff oder ein Gemisch davon.

16. Verwendung nach einem der Ansprüche 1 bis 12, worin das Medikament formuliert ist zur Behandlung des nasalen Epithels, Nasennebenhöhlen, wobei die Medikamente in der Form von Lösungen zur Zulieferung als Tropfen, durch Pipette oder Spritze an das nasale Epithel, die Nasennebenhöhlen, zur Irrigation der Nebenhöhlen, sind.

17. Medikament zur Verwendung bei der Behandlung von Atemwegserkrankungen, die **gekennzeichnet sind durch** geringe Mukoziliar-Clearance oder Mukostase, wie in einem der Ansprüche 1 bis 16 definiert.

18. Inhalationsvorrichtung, befüllt mit dem in Anspruch 17 definierten Medikament.

19. Inhalationsvorrichtung nach Anspruch 18, worin die Vorrichtung ein Dosierinhalator, ein Strahlvernebler oder Ultraschallvernebler ist.

**Revendications**

1. Utilisation d'un composé amidino de formule générale :

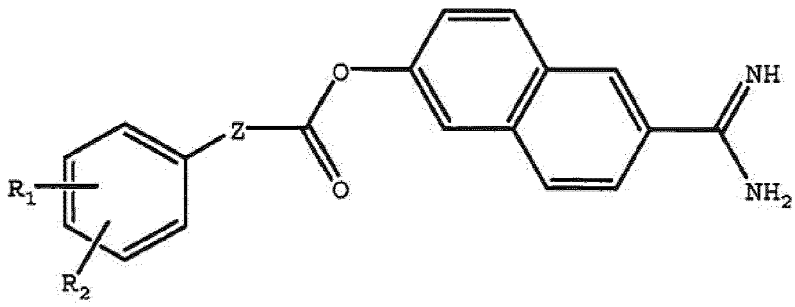

ou d'un sel pharmaceutiquement acceptable de celui-ci, dans la fabrication d'un médicament destiné au traitement de maladies respiratoires **caractérisées par** une mucostase ou une clairance muco-ciliaire médiocre, formule dans laquelle :

Z représente - $(CH_2)_a$-,

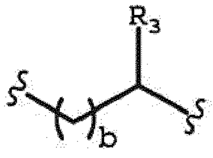 , 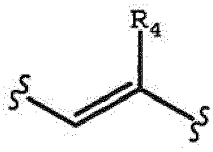 ou 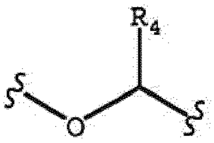

où a vaut 0, 1, 2 ou 3, b vaut 0, 1 ou 2, $R_3$ est un groupe alkyle linéaire ou ramifié ayant 1 à 4 atomes de carbone ou un groupe cycloalkyle ayant 3 à 6 atomes de carbone, $R_4$ est un atome d'hydrogène ou un groupe alkyle linéaire ou ramifié ayant 1 à 4 atomes de carbone ;
chacun de $R_1$ et $R_2$, qui peuvent être identiques ou différents, représente un atome d'hydrogène, un groupe alkyle linéaire ou ramifié ayant 1 à 4 atomes de carbone, -O- $R_5$, -S- $R_5$, -$COOR_5$, -$COR_6$, -O- $COR_7$, -$NHCOR_7$,

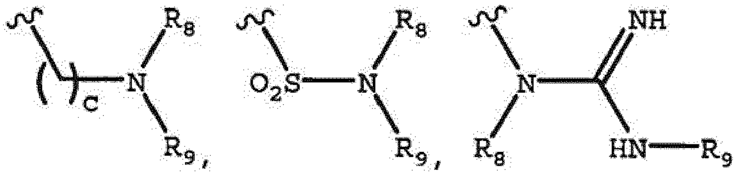

$NO_2$, CN, un halogène, $CF_3$, méthylènedioxy, ou

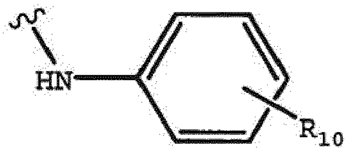

où c vaut 0, 1 ou 2 ; $R_5$ est un atome d'hydrogène, un groupe alkyle linéaire ou ramifié ayant 1 à 4 atomes de carbone, ou un groupe benzyle ; $R_6$ est un atome d'hydrogène ou un groupe alkyle linéaire ou ramifié ayant 1 à 4 atomes de carbone ; $R_7$ est un groupe alkyle linéaire ou ramifié ayant 1 à 4 atomes de carbone ; chacun de $R_8$ et $R_9$, qui peuvent être identiques ou différents, est un atome d'hydrogène, un groupe alkyle linéaire ou ramifié ayant 1 à 4 atomes de carbone, ou un groupe protecteur de radical amino ; et $R_{10}$ est un atome d'hydrogène, méthyle ou $CF_3$,

dans laquelle le médicament est une solution aqueuse ayant une concentration de chlorure inférieure à 10 mM.

2. Utilisation selon la revendication 1, dans laquelle le composé amidino est sous la forme d'un ester ou d'un sel pharmaceutiquement acceptable.

3. Utilisation selon l'une quelconque des revendications précédentes, dans laquelle le composé amidino est le 4-guanidinobenzoate de 6- amidino- 2- naphtyle.

4. Utilisation selon la revendication 3, dans laquelle le 4- guanidinobenzoate de 6- amidino- 2- naphtyle est sous la forme du dichlorhydrate de 4- guanidinobenzoate de 6- amidino- 2- naphtyle ou du mésylate de 4- guanidinobenzoate de 6- amidino- 2- naphtyle.

5. Utilisation selon l'une quelconque des revendications précédentes, dans laquelle le médicament comprend en outre un ou plusieurs diluants ou véhicules pharmaceutiquement acceptables.

6. Utilisation selon la revendication 5, dans laquelle le médicament comprend en outre un anion monovalent pharmaceutiquement acceptable n'ayant pas de capacité de perméation, à raison de 100 mM à 160 mM, de préférence à raison de 140 mM à 150 mM.

7. Utilisation selon la revendication 5, dans laquelle l'anion monovalent pharmaceutiquement acceptable n'ayant pas de capacité de perméation est le gluconate.

8. Utilisation selon l'une quelconque des revendications précédentes, dans laquelle le composé amidino est le seul agent actif.

9. Utilisation selon l'une quelconque des revendications 1 à 7, dans laquelle le médicament comprend aussi un ou plusieurs autres agents actifs choisis dans le groupe constitué par les antibiotiques, les vaccins, les décongestionnants (à usage nasal ou bronchique), les agents mucolytiques, la rhDNase, les anti-inflammatoires non stéroïdiens (AINS), les stéroïdes, les agents antiviraux, les inhibiteurs d'élastase, les agents bloqueurs des canaux sodiques exofaciaux, les agents de thérapie génique, les activateurs des canaux de chlorure, le mannitol, les agonistes des récepteurs purinergiques et les bronchodilatateurs.

10. Utilisation selon la revendication 9, dans laquelle le médicament comprend un seul agent actif additionnel.

11. Utilisation selon l'une quelconque des revendications précédentes, dans laquelle la maladie respiratoire est choisie dans le groupe comprenant la mucoviscidose (fibrose kystique, FK) ; la bronchiectasie ; la bronchite chronique ; la broncho-pneumopathie chronique obstructive (BPCO) et la rhino-sinusite.

12. Utilisation selon l'une quelconque des revendications précédentes, dans laquelle la maladie respiratoire est la mucoviscidose.

13. Utilisation selon l'une quelconque des revendications précédentes, dans laquelle le médicament est formulé pour une administration au système respiratoire par la voie pulmonaire.

14. Utilisation selon la revendication 13, dans laquelle le médicament est formulé pour une administration par l'une quelconque parmi une instillation intratrachéale, une délivrance intra-trachéale de liposomes, une insufflation, une nébulisation, et une inhalation d'aérosol.

15. Utilisation selon la revendication 14, dans laquelle le médicament est formulé pour une administration par inhalation d'aérosol, conjointement avec un propulseur choisi dans le groupe comprenant les hydrofluoroalcanes, les chlorofluorocarbures, le propane, l'azote, ou un de leurs mélanges.

16. Utilisation selon l'une quelconque des revendications 1 à 12, dans laquelle le médicament est formulé pour le traitement de l'épithélium nasal, des sinus aériens, ledit médicament étant sous la forme d'une solution pour délivrance sous la forme de gouttes, au moyen d'une pipette ou au moyen d'une seringue à l'épithélium nasal ou aux sinus aériens, pour l'irrigation des sinus aériens.

17. Médicament pour une utilisation dans le traitement de maladies respiratoires **caractérisées par** une mucostase ou

une clairance muco-ciliaire médiocre, telle que définie dans l'une quelconque des revendications 1 à 16.

**18.** Dispositif d'inhalation chargé du médicament tel que défini dans la revendication 17.

**19.** Dispositif d'inhalation selon la revendication 18, lequel dispositif est un inhalateur doseur, un nébuliseur à jet ou un nébuliseur à ultrasons.

**Figure 1**

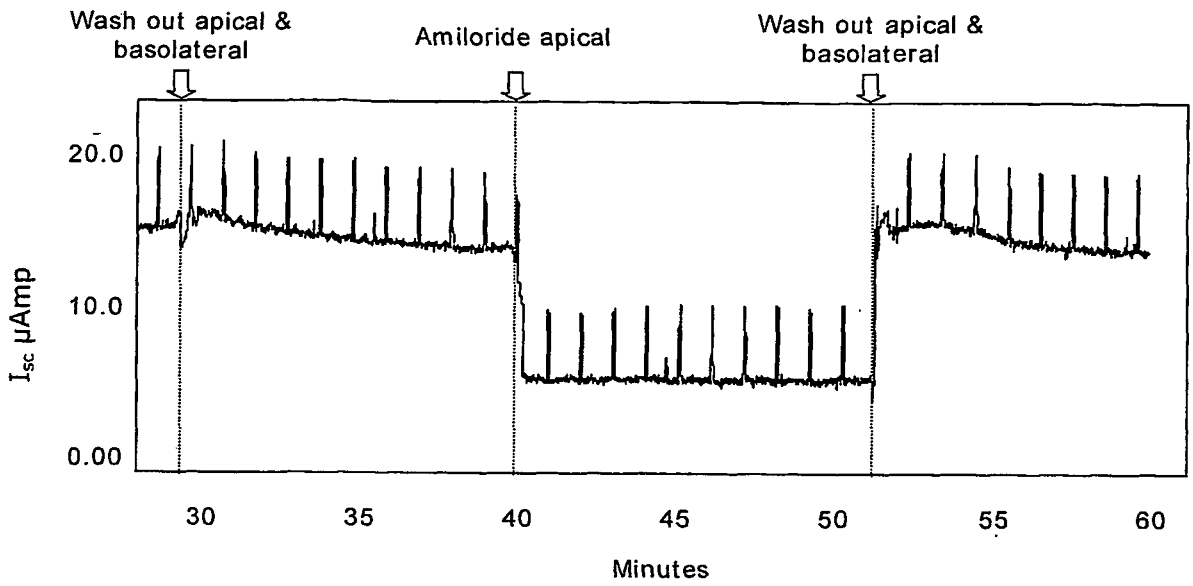
Wash out apical & basolateral
Amiloride apical
Wash out apical & basolateral
20.0
10.0
0.00
$I_{sc}$ µAmp
30
35
40
45
50
55
60
Minutes

**Figure 2**

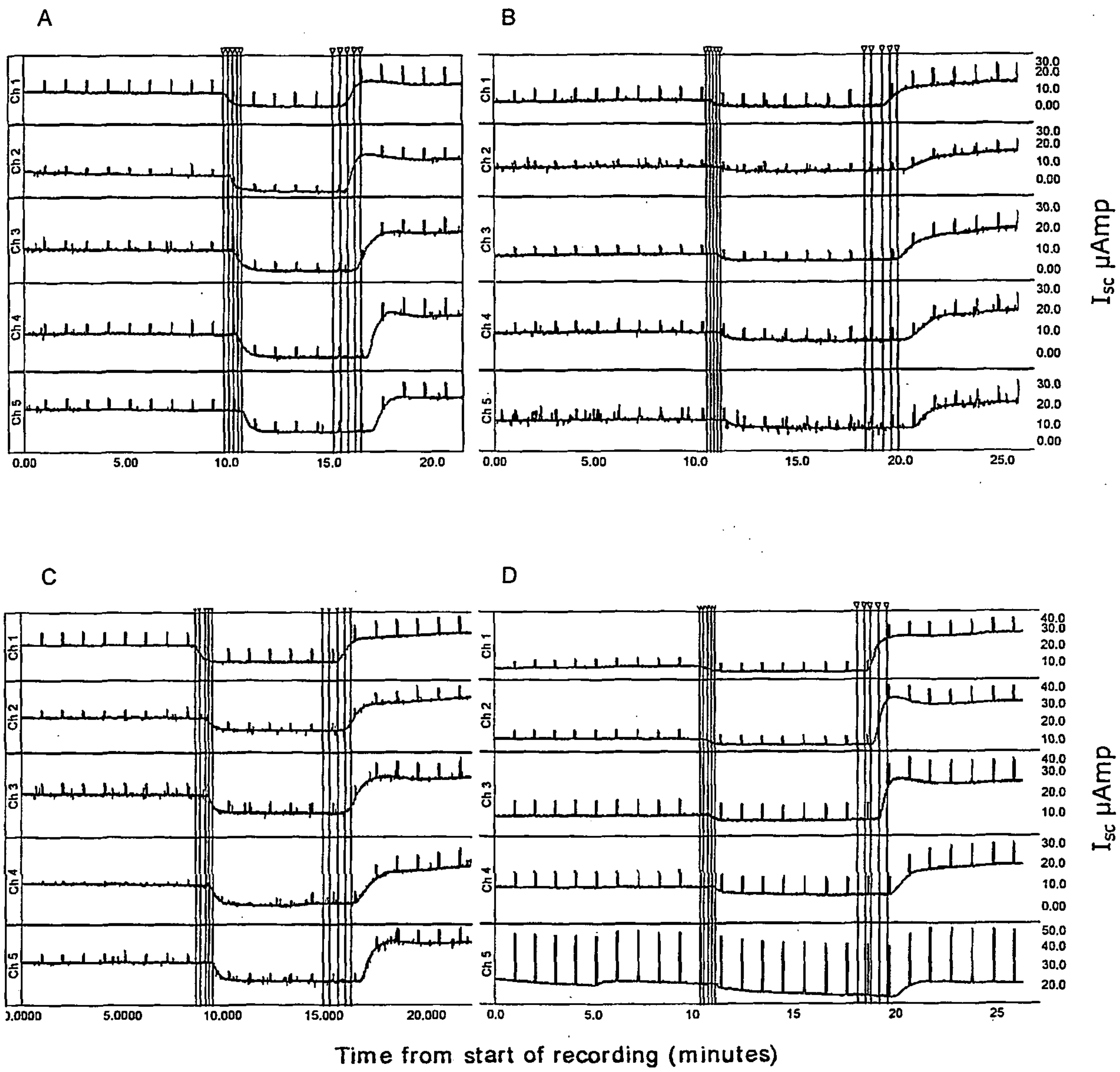
A
B
C
D
Ch 1
Ch 2
Ch 3
Ch 4
Ch 5
Isc μAmp
Time from start of recording (minutes)

**Figure 3**

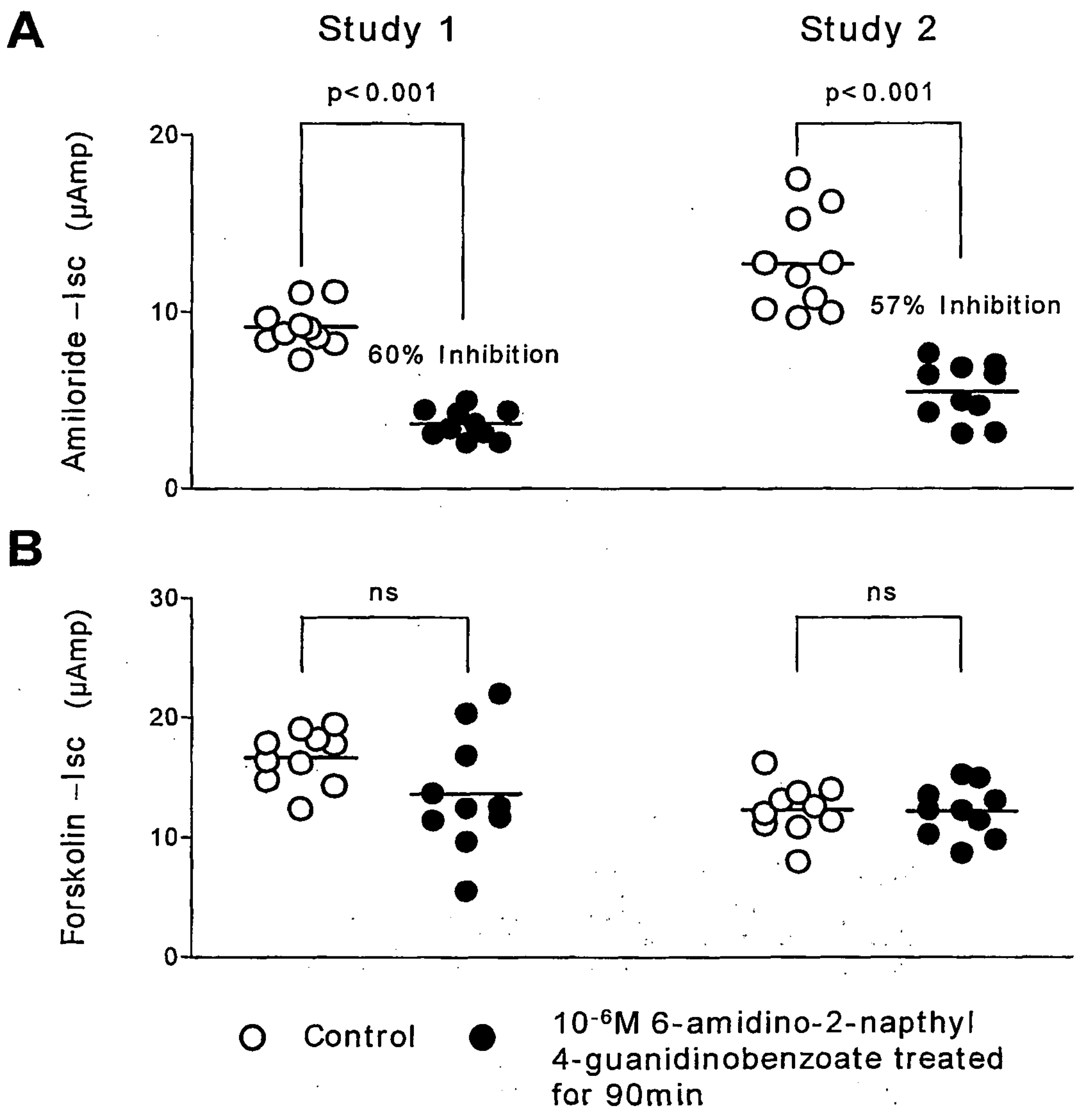
A
Study 1
Study 2
p<0.001
p<0.001
Amiloride -Isc (µAmp)
20
10
0
60% Inhibition
57% Inhibition
B
Forskolin -Isc (µAmp)
30
20
10
0
ns
ns
Control
10-6M 6-amidino-2-napthyl 4-guanidinobenzoate treated for 90min

**Figure 4**

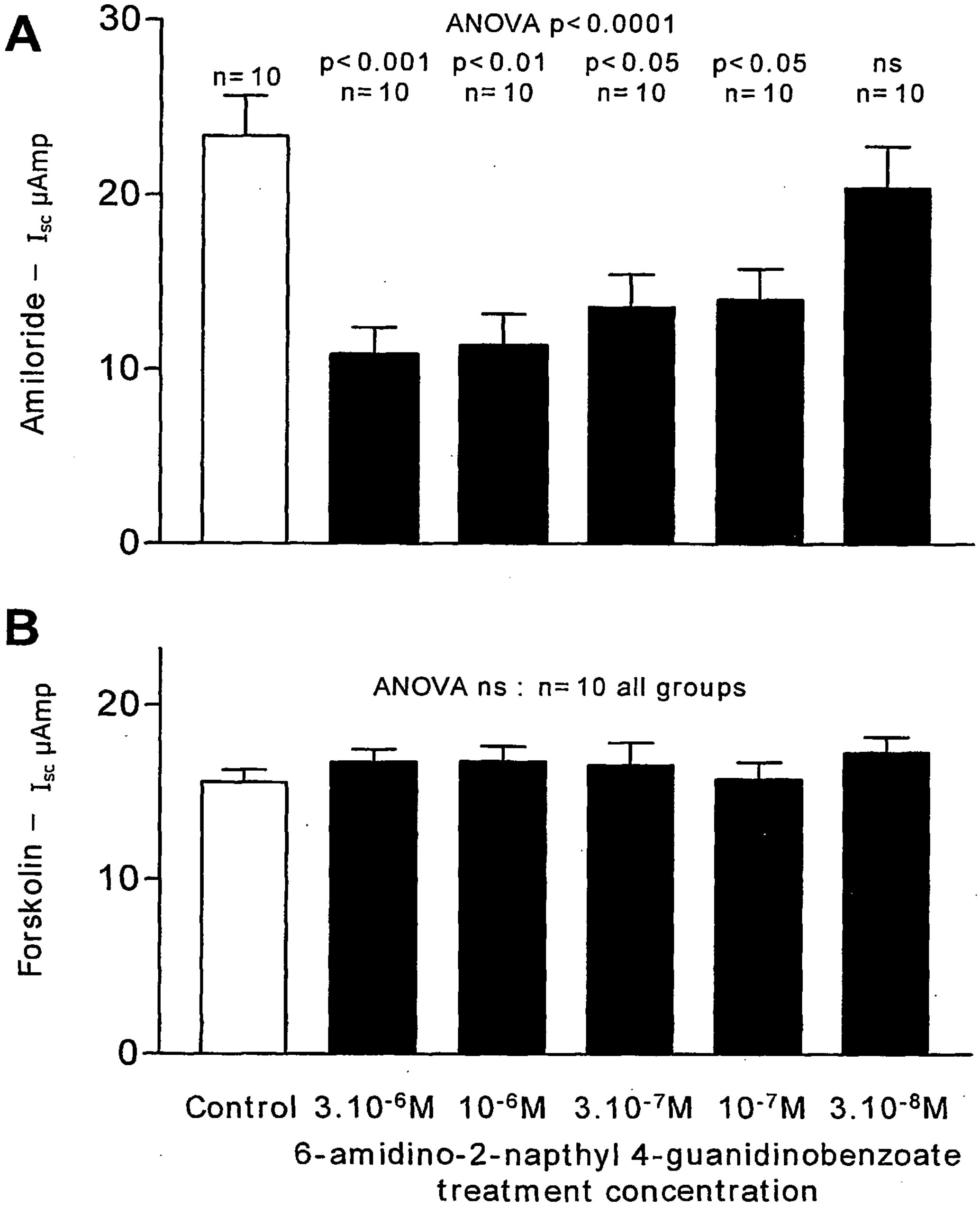
A
30
20
10
0
Amiloride - Isc µAmp
ANOVA p< 0.0001
n= 10
p< 0.001
n= 10
p< 0.01
n= 10
p< 0.05
n= 10
p< 0.05
n= 10
ns
n= 10
B
20
10
0
Forskolin - Isc µAmp
ANOVA ns : n= 10 all groups
Control 3.10-6M 10-6M 3.10-7M 10-7M 3.10-8M
6-amidino-2-napthyl 4-guanidinobenzoate
treatment concentration

**Figure 5**

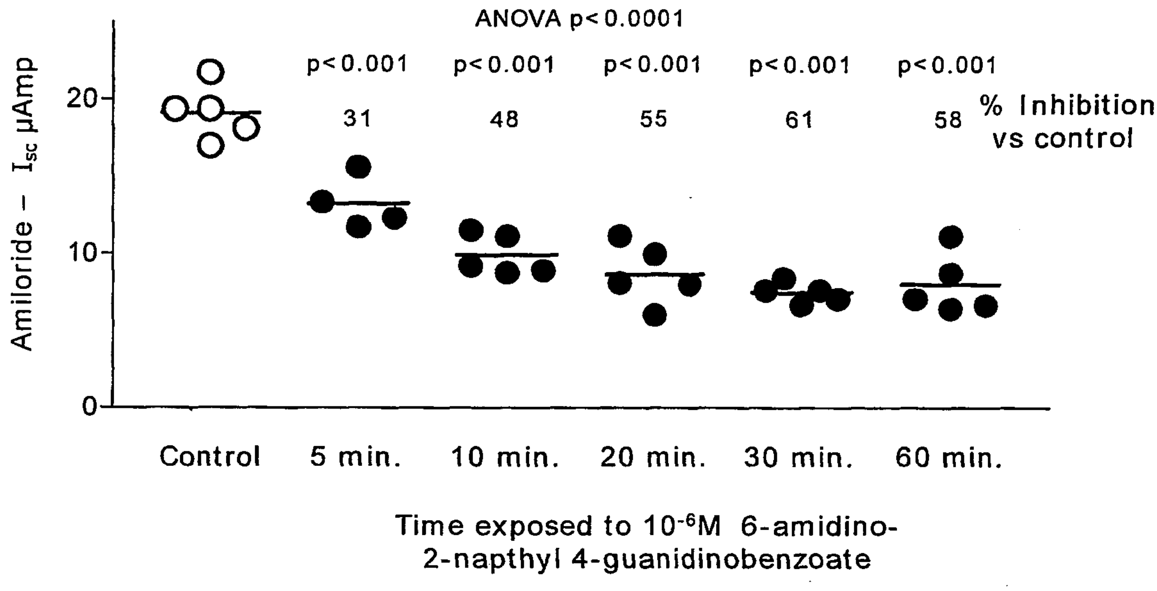
ANOVA p<0.0001
p<0.001
p<0.001
p<0.001
p<0.001
p<0.001
31
48
55
61
58
% Inhibition vs control
Amiloride - $I_{sc}$ μAmp
20
10
0
Control
5 min.
10 min.
20 min.
30 min.
60 min.
Time exposed to $10^{-6}$M 6-amidino-2-napthyl 4-guanidinobenzoate

**Figure 6**

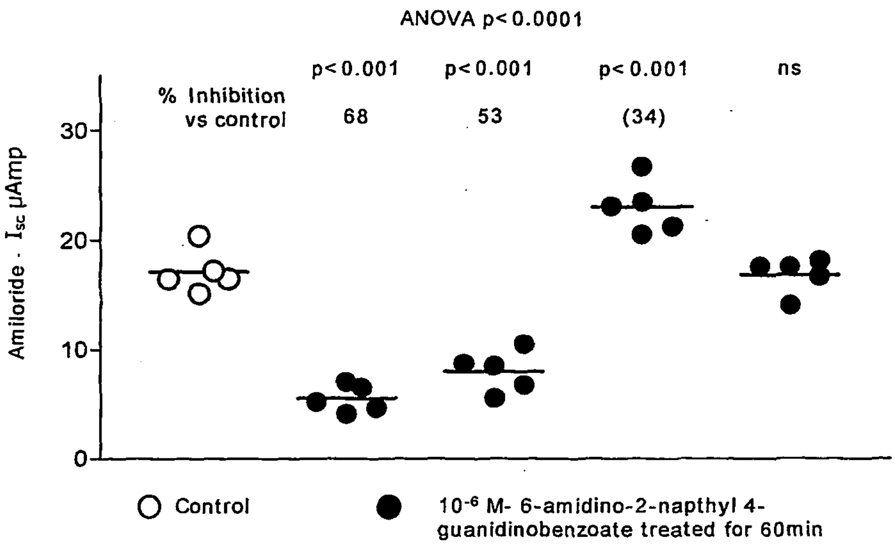


| | | | | | |
|---|---|---|---|---|---|
| Washout period fresh culture medium | 0min. | 0min. | 1h15min. | 3h00min. | 4h30min. |
| Time in KR buffer prior to amiloride addition | 30min. | 30min. | 30min. | 30min. | 30min. |
| Total time post exposure to 6-amidino-2-napthyl 4-guanidinobenzoate prior to amiloride addition | - | 30min. | 1h45min. | 3h30min. | 5h00min. |

**Figure 7**

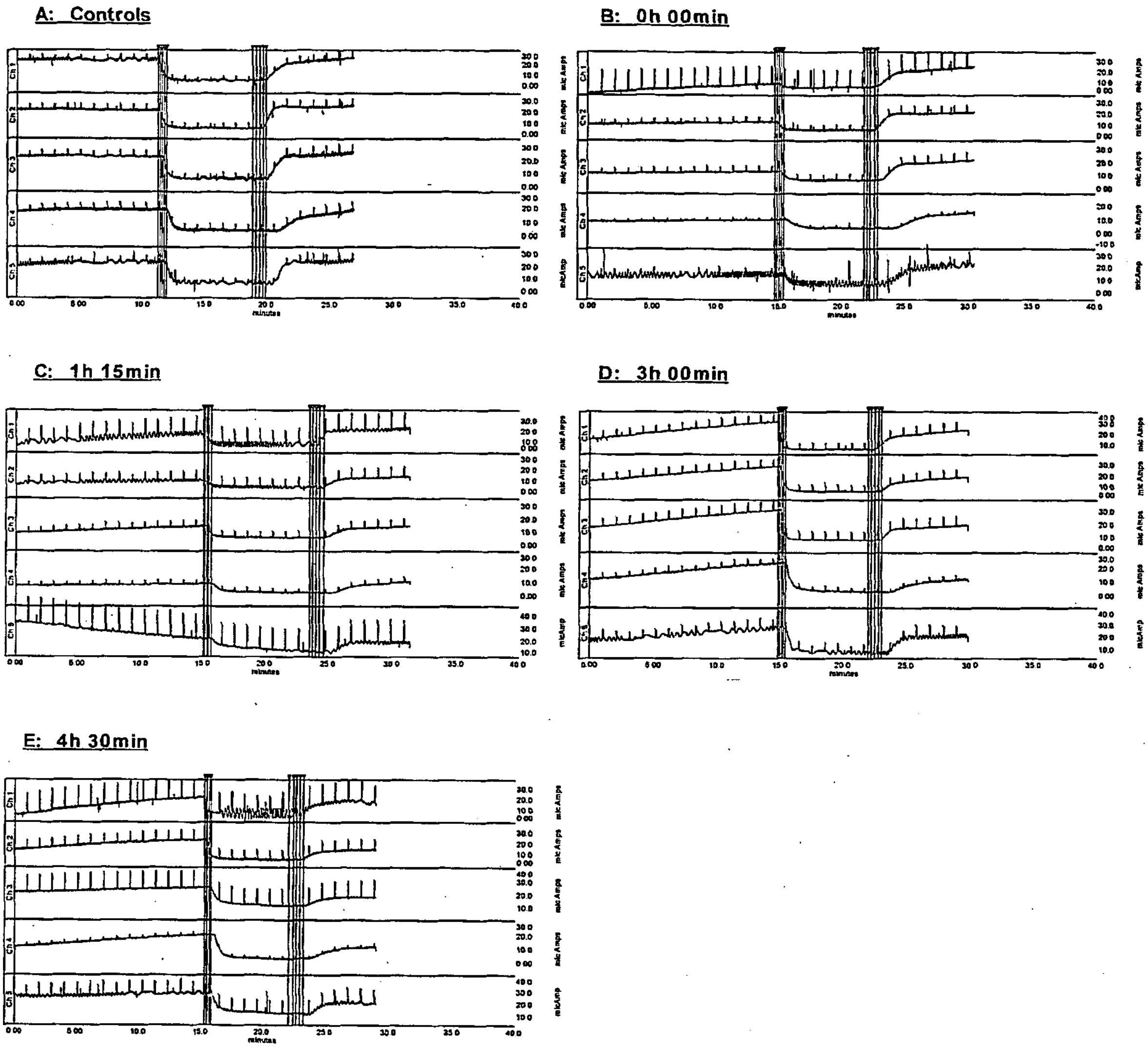

**Figure 8**

A

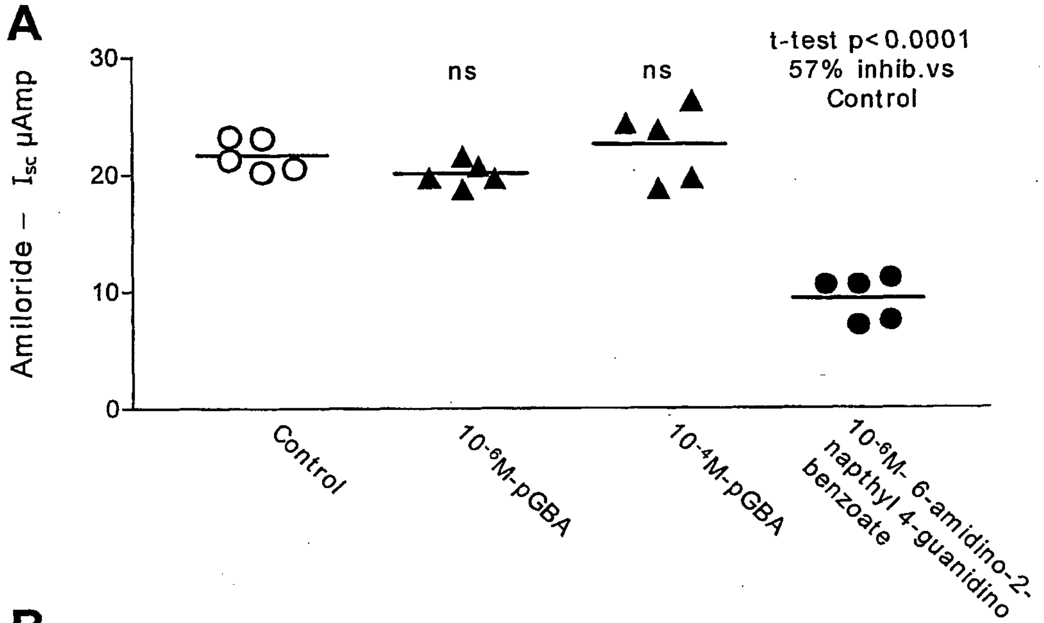
t-test p<0.0001
57% inhib.vs
Control
ns
ns
Amiloride – Isc µAmp
30
20
10
0
Control
10-6M-pGBA
10-4M-pGBA
10-6M- 6-amidino-2-napthyl 4-guanidino benzoate

B

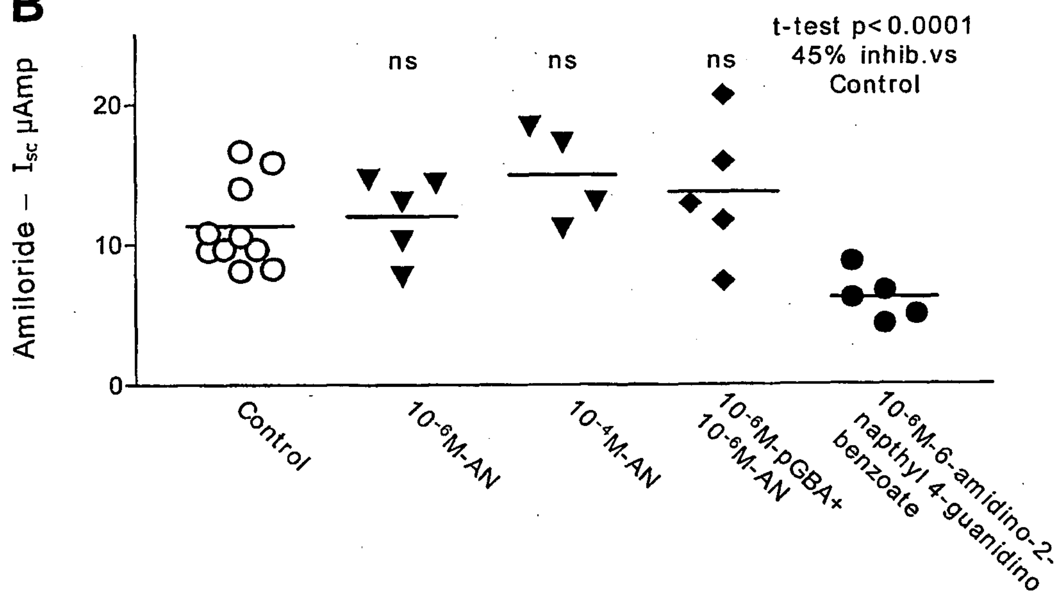
t-test p<0.0001
45% inhib.vs
Control
ns
ns
ns
Amiloride – Isc µAmp
20
10
0
Control
10-6M-AN
10-4M-AN
10-6M-pGBA+
10-6M-AN
10-6M-6-amidino-2-napthyl 4-guanidino benzoate

**Figure 9:**

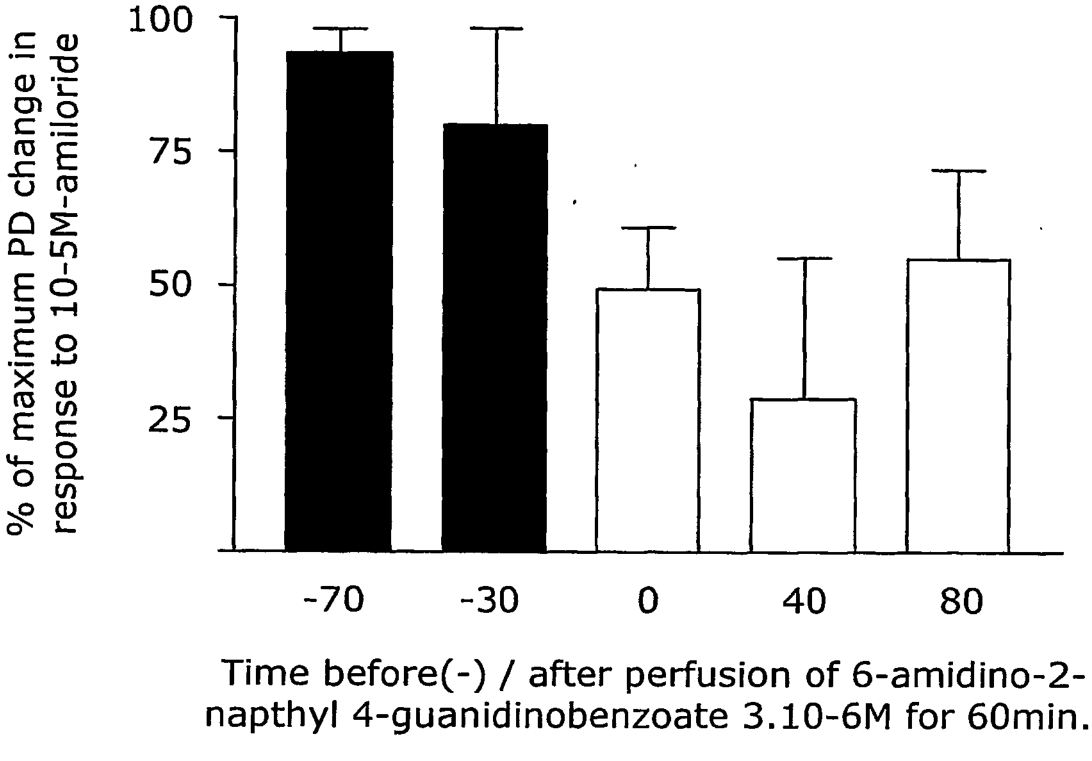
% of maximum PD change in response to 10-5M-amiloride
100
75
50
25
-70
-30
0
40
80
Time before(-) / after perfusion of 6-amidino-2-napthyl 4-guanidinobenzoate 3.10-6M for 60min.

**Figure 10**

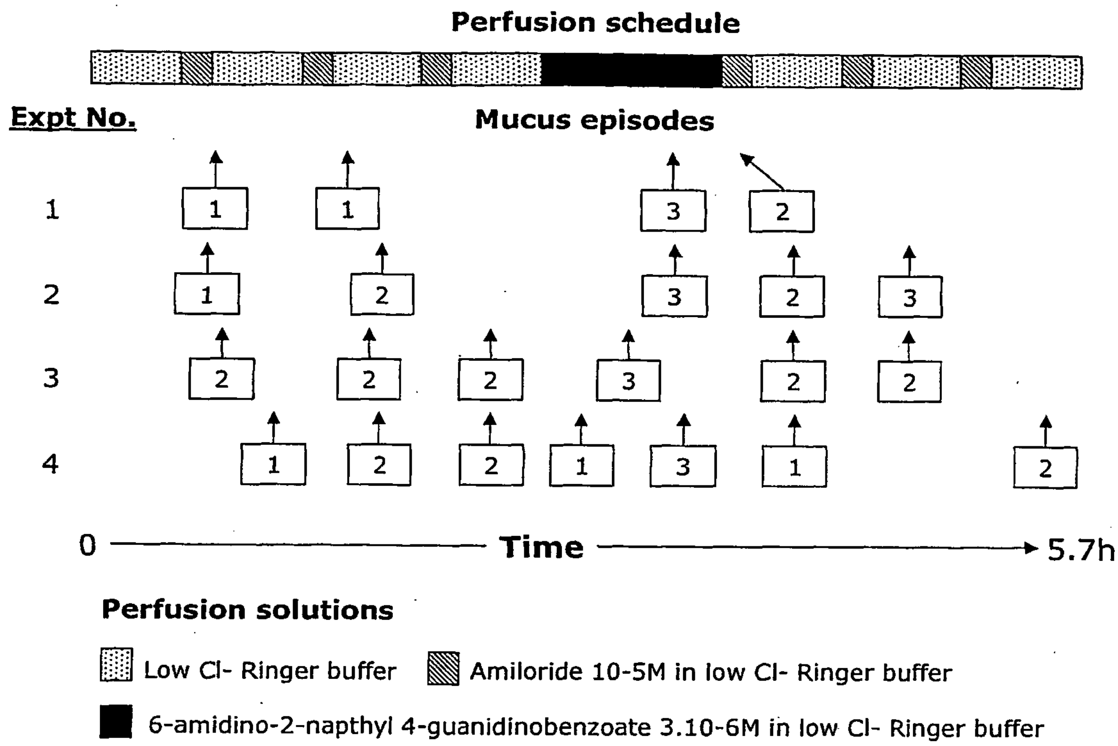
Perfusion schedule
Expt No.
Mucus episodes
1
2
3
4
1 1 3 2
1 2 3 2 3
2 2 2 3 2 2
1 2 2 1 3 1 2
0
Time
5.7h
Perfusion solutions
Low Cl- Ringer buffer
Amiloride 10-5M in low Cl- Ringer buffer
6-amidino-2-napthyl 4-guanidinobenzoate 3.10-6M in low Cl- Ringer buffer

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 57053454 B
- US 4454338 A
- EP 1314779 A

### Non-patent literature cited in the description


- **BRIDGES RJ ; NEWTON BB ; PILEWSKI JM ; DEVOR DC ; POLL CT. ; HALL RL.** Na+ transport in normal and CF human bronchial epithelial cells is inhibited by BAY 39-9437. *Am J Physiol Lung Cell Mol Physiol.,* 2001, vol. 281, L16-23
- **DANAHAY H ; ATHERTON H ; JONES G ; BRIDGES RJ. ; POLL CT.** Interleukin-13 induces a hypersecretory ion transport phenotype in human bronchial epithelial cells. *Am J Physiol Lung Cell Mol Physiol.,* 2002, vol. 282, L226-236
- **HITOMI Y ; IKARI N ; FUJII S.** Inhibitory effect of a new synthetic protease inhibitor (FUT-175) on the coagulation system. *Haemostasis,* 1985, vol. 15, 164-8
- **IWAKI M ; ODA M ; OZEKI M ; INO Y ; SUZUKI K ; KOSHIYAMA Y ; MOTOYOSHI A ; OGIHARA M ; SUZUKI S ; FUJITA M et al.** Pharmacological studies of FUT-175, nafamstat mesilate. III. Anti-inflammatory activities of FUT-175. *Nippon Yakurigaku Zasshi,* 1984, vol. 84, 373-84
- **IWASHITA K ; KITAMURA K ; NARIKIYO T ; ADACHI M ; SHIRAISHI N ; MIYOSHI T ; NAGANO J ; TUYEN DG ; NONOGUCHI H. ; TOMITA K.** Inhibition of prostasin secretion by serine protease inhibitors in the kidney. *J Am Soc Nephrol.,* 2003, vol. 14, 11-16
- **MUTO, S ; IMAI M. ; ASANO Y.** Effect of nafamostat mesilate on Na+ and K+ transport properties in the rabbit cortical collecting duct. *Br. J. Pharmacol.,* 1993, vol. 109, 673-678
- **MUTO S ; IMAI M. ; ASANO Y.** Mechanisms of the hyperkalaemia caused by nafamostat mesilate: effects of its two metabolites on Na+ and K+ transport properties in the rabbit cortical collecting duct. *Br.J.Pharmacol.,* 1994, vol. 111, 173-178
- **NARIKIYO T ; KITAMURA K ; ADACHI M ; MIYOSHI T ; IWASHITA K ; SHIRAISHI N ; NONOGUCHI H ; CHEN LM ; CHAI KX ; CHAO J.** Regulation of prostasin by aldosterone in the kidney. *J Clin Invest.,* 2002, vol. 109, 401-408
- **ROSSIER BC.** The epithelial sodium channel: activation by membrane-bound serine proteases. *Proc Am Thorac Soc.,* 2004, vol. 1, 4-9
- Standardized procedure for measurement of nasal potential difference: an outcome measure in multicenter cystic fibrosis clinical trials. *Pediatr Pulmonol.,* 2004, vol. 37 (5), 385-92
- **TONG Z ; ILLEK B ; BHAGWANDIN VJ ; VERGHESE GM. ; CAUGHEY GH.** Prostasin, a membrane-anchored serine peptidase, regulates sodium currents in JME/CF15 cells, a cystic fibrosis airway epithelial cell line. *Am J Physiol Lung Cell Mol Physiol.,* 2004, vol. 287, L928-935
- **VALLET V ; CHRAIBI A ; GAEGGELER HP ; HORISBERGER JD ; ROSSIER BC.** An epithelial serine protease activates the amiloride-sensitive sodium channel. *Nature,* 1997, vol. 389, 607-610